# EUROPEAN PATENT SPECIFICATION

(11) **EP 4 429 561 B1**
(45) Date of publication and mention of the grant of the patent: **26.02.2025**
(21) Application number: 22839019.1
(22) Date of filing: 14.11.2022
(51) Int. Cl.: A61B 8/08, A61B 8/00, G10K 11/34, G01S 7/52, G01S 15/89

(54) **METHOD AND SYSTEM FOR ADJUSTING SCAN PATTERN FOR ULTRASOUND IMAGING**
VERFAHREN UND SYSTEM ZUR ANPASSUNG EINES ABTASTMUSTERS FÜR ULTRASCHALLBILDGEBUNG
PROCÉDÉ ET SYSTÈME DE RÉGLAGE DE MOTIF DE BALAYAGE POUR IMAGERIE ULTRASONORE

(30) Priority: 12.11.2021 US 202163278981 P; 16.12.2021 US 202163290243 P
(43) Date of publication of application: 18.09.2024
(73) Proprietor: BFLY Operations, Inc., Burlington, MA 01803 (US)
(72) Inventor: NEBEN, Abraham, Burlington, MA 01803 (US); SANCHEZ, Nevada, J., Burlington, MA 01803 (US); MAHER, Patrick, T., Burlington, MA 01803 (US); THIELE, Karl, Burlington, MA 01803 (US); OFFERDAHL, Katelyn, Burlington, MA 01803 (US)
(74) Representative: Kelly, Edward
(86) International application number: PCT/US2022/049751
(87) International publication number: WO 2023/086605

(56) References cited:
- US-A1- 2003 092 994
- US-A1- 2004 044 284
- US-A1- 2011 016 977
- US-A1- 2021 137 498

## Description

### BACKGROUND

Ultrasound imaging has a wide range of applications in the medical and scientific fields for diagnosis, treatment, and studying of internal objects, within a body, such as internal organs or developing fetuses. An ultrasound probe typically includes an array of transducers that transmit and receive ultrasound signals that are used for these imaging techniques.

US 2021/0137498 A1 describes an ultrasound imaging system configured to identify a scan line pattern for imaging an object or feature thereof. The system may include a controller that controls a probe for imaging a volume of a subject by transmitting and receiving ultrasound signals in accordance with a plurality of scan line patterns. One or more processors communicating with the probe may generate a plurality of image data sets based on the signals received at the probe, each data set corresponding to a discrete scan line pattern. These data sets are assessed for a target characteristic specific to the object targeted for imaging. One the data set that includes the target characteristic is identified, the one or more processors select the scan line pattern that corresponds the identified image data set. This scan line pattern may then be used for subsequent imaging of the volume to view the object.

### SUMMARY

This summary is provided to introduce a selection of concepts that are further described below in the detailed description. This summary is not intended to identify key or essential features of the claimed subject matter, nor is it intended to be used as an aid in limiting the scope of the claimed subject matter.

A system according to the invention is defined in independent claim 1, a method according to the invention is defined in independent claim 9.

Other aspects of the disclosure will be apparent from the dependent claims and the following description.

### BRIEF DESCRIPTION OF DRAWINGS

FIG. 1A shows an ultrasound system in accordance with one or more embodiments.
FIG. 1B shows a block diagram of an ultrasound device in accordance with one or more embodiments.
FIG. 2 shows an example ultrasound system in accordance with one or more embodiments.
FIG. 3A shows a phased array in accordance with one or more embodiments.
FIGs. 3B-3E show an operation of a phased array in accordance with one or more embodiments.
FIG. 4A shows an azimuthal aperture translation in accordance with one or more embodiments.
FIG. 4B shows an azimuthal tilt in accordance with one or more embodiments.
FIGs. 4C and 4D show an elevational tilt in accordance with one or more embodiments.
FIGs. 5A and 5B shows a flowchart of a method in accordance with one or more embodiments.
FIGs. 6A and 6B show ultrasound imaging of a lung in accordance with one or more embodiments.
FIGs. 7A and 7B show cardiac ultrasound imaging.
FIG. 7C shows cardiac ultrasound imaging in accordance with one or more embodiments.
FIGs. 8A-8D illustrate a sequence of ultrasound image frames taken of a subject's heart in accordance with one or more embodiments.
FIG. 9A shows an example handheld ultrasound probe, in accordance with one or more embodiments.
FIG. 9B shows an example wearable ultrasound patch, in accordance with one or more embodiments.

### DETAILED DESCRIPTION

Conventional ultrasound systems are large, complex, and expensive systems that are typically only purchased by large medical facilities with significant financial resources. Recently, less expensive and less complex ultrasound imaging devices have been introduced. Such devices may include ultrasonic transducers monolithically integrated onto a single semiconductor die to form a monolithic ultrasound device. Aspects of such ultrasound-on-a chip devices are described in U.S. Patent Application No. 15/415,434 titled "UNIVERSAL ULTRASOUND DEVICE AND RELATED APPARATUS AND METHODS," filed on January 25, 2017 (and assigned to the assignee of the instant application), published as U.S. Pat. Pub. No. 2017/0360397 Al and issued as U.S. Pat. No. 10,856,840 (the '840 patent). The reduced cost and increased portability of these new ultrasound devices may make them significantly more accessible to the general public than conventional ultrasound devices. The inventors have recognized and appreciated that although the reduced cost and increased portability of some ultrasound imaging devices, such as those described in the '840 patent, makes them more accessible to the general populace, people who could make use of such devices have little to no training for how to use them. Ultrasound examinations often include the acquisition of ultrasound images that contain a view of a particular anatomical structure (e.g., an organ) of a subject. Acquisition of these ultrasound images typically requires considerable skill.

In general, embodiments of the disclosure provide a method, non-transitory computer readable medium (CRM), and system assisting in acquiring clinically usable ultrasound images. Embodiments of the disclosure provide technology for automatically adjusting a scan pattern to obtain a desired or target view of a specified anatomy without user intervention. In this manner, the ultrasound imaging devices may be productively employed even when the user is unable to reliably position or adjust the device to get the target view on their own.

This technique may be particularly helpful, for example, for cardiac imaging because the "cardiac windows" through the ribs and lungs can be hard to find, and may vary from patient to patient. Fine probe movements are typically required to find the correct view, and novices often struggle to obtain acceptable cardiac images or lung images. However, auto-steering could also be helpful for many other clinical applications. For example, auto-steering may be used to image organs such as the lungs, carotid, kidney, aorta, thyroid, bladder, etc., thus finding the best view (in the best preset) of the anatomy. Some embodiments of the present application may utilize or benefit from use of an ultrasound probe of the types described in the '840 patent, referenced above. An ultrasound probe having a 2D aperture with electronically controllable scan pattern (e.g., controllable in the azimuth, elevation, both, other in other manners) may facilitate automatic adjustment of the scanning and beneficial capture of target images. A detailed description is subsequently provided.

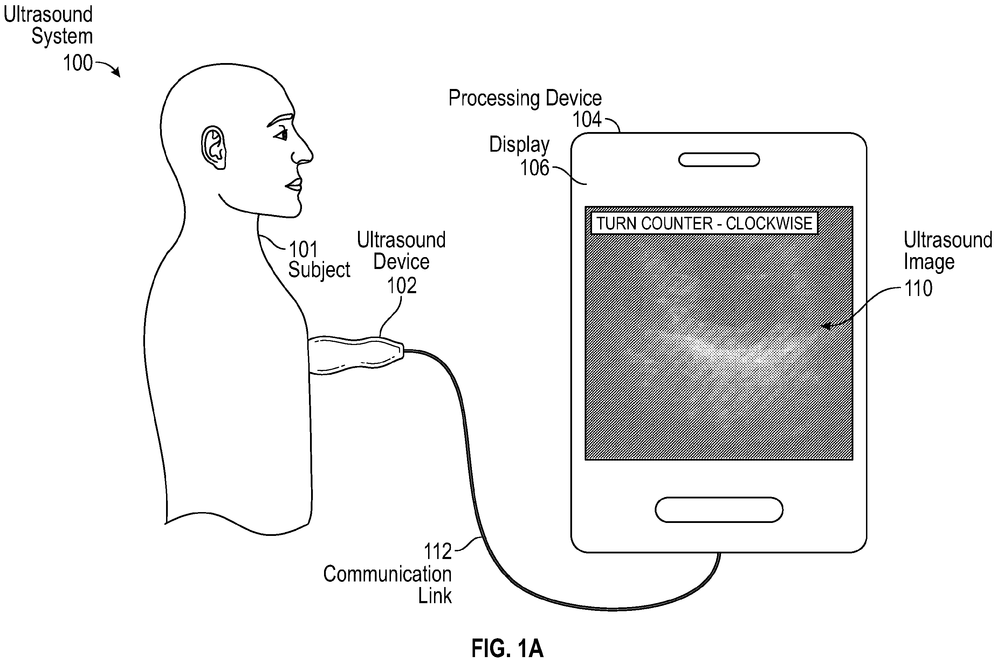

FIG. 1A shows an example ultrasound system (100) including an ultrasound device (102) configured to obtain an ultrasound image of a target anatomical view of a subject (101). As shown, the ultrasound system (100) comprises an ultrasound device (102) that is communicatively coupled to the processing device (104) by a communication link (112). The processing device (104) may be configured to receive ultrasound data from the ultrasound device (102) and use the received ultrasound data to generate an ultrasound image (110) on a display (which may be touch-sensitive) of the processing device (104). In some embodiments, the processing device (104) provides the operator with instructions (e.g., images, videos, or text) prior to the operator scanning the subject (101). The processing device (104) may provide quality indicators and/or labels of anatomical features during scanning of the subject (101) to assist a user in collecting clinically relevant ultrasound images.

The ultrasound device (102) may be configured to generate ultrasound data. The ultrasound device (102) may be configured to generate ultrasound data by, for example, emitting acoustic waves into the subject (101) and detecting the reflected acoustic waves. The detected reflected acoustic wave may be analyzed to identify various properties of the tissues through which the acoustic wave traveled, such as a density of the tissue. The ultrasound device (102) may be implemented in any of a variety of ways. For example, the ultrasound device (102) may be implemented as a handheld device (as shown in FIG. lA) or as a patch that is coupled to patient using, for example, an adhesive.

The ultrasound device (102) may transmit ultrasound data to the processing device 104 using the communication link (112). The communication link (112) may be a wired or wireless communication link. In some embodiments, the communication link (112) may be implemented as a cable such as a Universal Serial Bus (USB) cable or a Lightning cable. In these embodiments, the cable may also be used to transfer power from the processing device 104 to the ultrasound device (102). In other embodiments, the communication link (112) may be a wireless communication link such as a BLUETOOTH, WiFi, or ZIGBEE wireless communication link.

The processing device (104) may comprise one or more processing elements (such as a processor) to, for example, process ultrasound data received from the ultrasound device (102). Additionally, the processing device (104) may comprise one or more storage elements (such as a non-transitory computer readable medium) to, for example, store instructions that may be executed by the processing element(s) and/or store all or any portion of the ultrasound data received from the ultrasound device (102). It should be appreciated that the processing device (104) may be implemented in any of a variety of ways. For example, the processing device (104) may be implemented as a mobile device (e.g., a mobile smartphone, a tablet, or a laptop) with an integrated display (106) as shown in FIG. 1A. In other examples, the processing device (104) may be implemented as a stationary device such as a desktop computer.

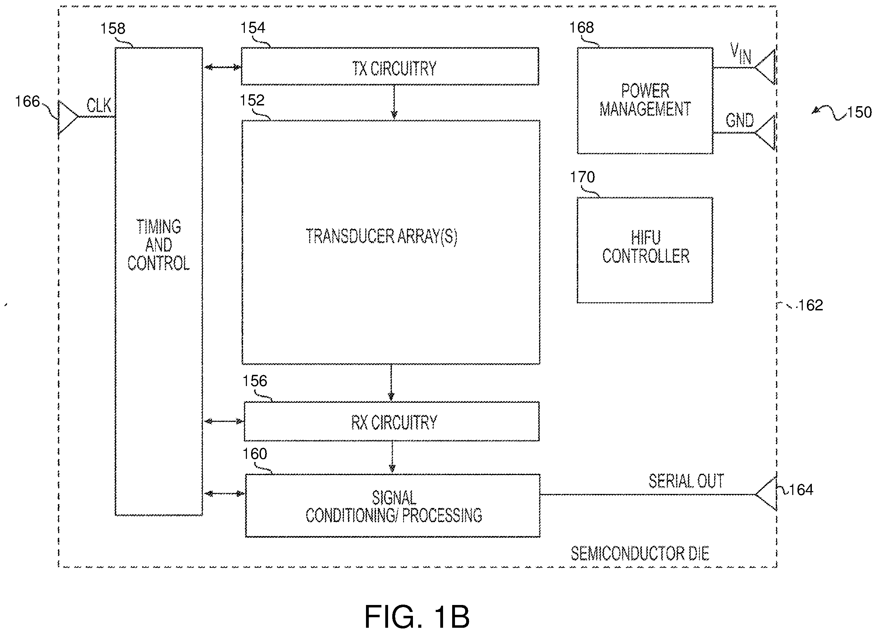

FIG. 1B is a block diagram of an example of an ultrasound device in accordance with some embodiments of the technology described herein. The illustrated ultrasound device (150) may include one or more ultrasonic transducer arrangements (e.g., arrays) (152), transmit (TX) circuitry (154), receive (RX) circuitry (156), a timing and control circuit (158), a signal conditioning/processing circuit (160), and/or a power management circuit (168).

The one or more ultrasonic transducer arrays (152) may take on any of numerous forms, and aspects of the present technology do not necessarily require the use of any particular type or arrangement of ultrasonic transducer cells or ultrasonic transducer elements. For example, multiple ultrasonic transducer elements in the ultrasonic transducer array (152) may be arranged in one-dimension, or two-dimensions. Although the term "array" is used in this description, it should be appreciated that in some embodiments the ultrasonic transducer elements may be organized in a non-array fashion. In various embodiments, each of the ultrasonic transducer elements in the array (152) may, for example, include one or more capacitive micromachined ultrasonic transducers (CMUTs), or one or more piezoelectric micromachined ultrasonic transducers (PMUTs).

In a non-limiting example, the ultrasonic transducer array 152 may include between approximately 6,000-10,000 (e.g., 8,960) active CMUTs on the chip, forming an array of hundreds of CMUTs by tens of CMUTs (e.g., 140 × 64). The CMUT element pitch may be between 150-250 um, such as 208 um, and thus, result in the total dimension of between 10-50mm by 10-50mm (e.g., 29.12 mm × 13.312 mm).

In some embodiments, the TX circuitry (154) may, for example, generate pulses that drive the individual elements of, or one or more groups of elements within, the ultrasonic transducer array(s) (152) so as to generate acoustic signals to be used for imaging. The RX circuitry (156), on the other hand, may receive and process electronic signals generated by the individual elements of the ultrasonic transducer array(s) (152) when acoustic signals impinge upon such elements.

With further reference to FIG. 1B, in some embodiments, the timing and control circuit (158) may be, for example, responsible for generating all timing and control signals that are used to synchronize and coordinate the operation of the other elements in the ultrasound device (150). In the example shown, the timing and control circuit (158) is driven by a single clock signal CLK supplied to an input port (166). The clock signal CLK may be, for example, a highfrequency clock used to drive one or more of the on-chip circuit components. In some embodiments, the clock signal CLK may, for example, be a 1.5625GHz or 2.5GHz clock used to drive a high-speed serial output device (not shown in FIG. 1B) in the signal conditioning/processing circuit (160), or a 20Mhz or 40 MHz clock used to drive other digital components on the die (162), and the timing and control circuit (158) may divide or multiply the clock CLK, as necessary, to drive other components on the die (162). In other embodiments, two or more clocks of different frequencies (such as those referenced above) may be separately supplied to the timing and control circuit (158) from an off-chip source.

In some embodiments, the output range of a same (or single) transducer unit in an ultrasound device may be anywhere in a range of 1-12 MHz (including the entire frequency range from 1-12 MHz), making it a universal solution, in which there is no need to change the ultrasound heads or units for different operating ranges or to image at different depths within a patient. That is, the transmit and/or receive frequency of the transducers of the ultrasonic transducer array may be selected to be any frequency or range of frequencies within the range of 1 MHz-12 MHz. The universal ultrasound device (150) described herein may thus be used for a broad range of medical imaging tasks including, but not limited to, imaging a patient's liver, kidney, heart, bladder, thyroid, carotid artery, lower venous extremity, and performing central line placement. Multiple conventional ultrasound probes would have to be used to perform all these imaging tasks. By contrast, a single universal ultrasound device (150) may be used to perform all these tasks by operating, for each task, at a frequency range appropriate for the task, as shown in the examples of Table 1 together with corresponding depths at which the subject may be imaged.

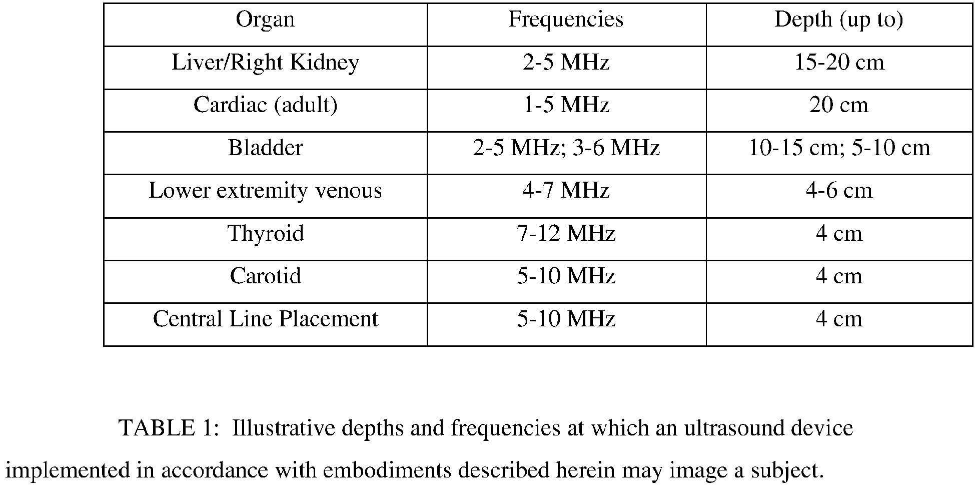

The power management circuit (168) may be, for example, responsible for converting one or more input voltages VIN from an off-chip source into voltages needed to carry out operation of the chip, and for otherwise managing power consumption within the ultrasound device (150). In some embodiments, for example, a single voltage (e.g., 12V, 80V, 100V, 120V, etc.) may be supplied to the chip and the power management circuit (168) may step that voltage up or down, as necessary, using a charge pump circuit or via some other DC-to-DC voltage conversion mechanism. In other embodiments, multiple different voltages may be supplied separately to the power management circuit (168) for processing and/or distribution to the other on-chip components.

In the embodiment shown above, all of the illustrated elements are formed on a single semiconductor die (162). It should be appreciated, however, that in alternative embodiments one or more of the illustrated elements may be instead located off-chip, in a separate semiconductor die (162), or in a separate device. Alternatively, one or more of these components may be implemented in a DSP chip, a field programmable gate array (FPGA) in a separate chip, or a separate application specific integrated circuity (ASIC) chip. Additionally, and/or alternatively, one or more of the components in the beamformer may be implemented in the semiconductor die (162), whereas other components in the beamformer may be implemented in an external processing device in hardware or software, where the external processing device is capable of communicating with the ultrasound device (150).

In addition, although the illustrated example shows both TX circuitry (154) and RX circuitry (156), in alternative embodiments only TX circuitry or only RX circuitry may be employed. For example, such embodiments may be employed in a circumstance where one or more transmission-only devices are used to transmit acoustic signals and one or more reception-only devices are used to receive acoustic signals that have been transmitted through or reflected off of a subject being ultrasonically imaged.

It should be appreciated that communication between one or more of the illustrated components may be performed in any of numerous ways. In some embodiments, for example, one or more high-speed busses (not shown), such as that employed by a unified Northbridge, may be used to allow high-speed intra-chip communication or communication with one or more off-chip components.

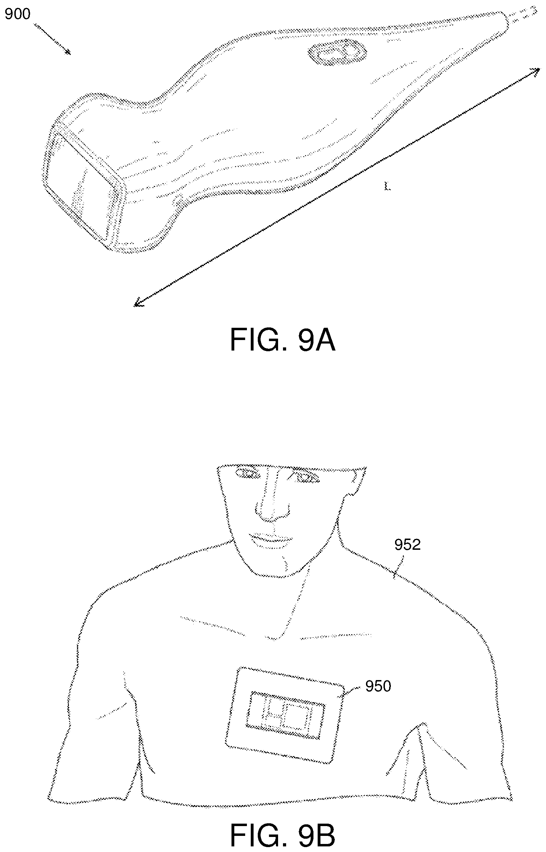

In some embodiments, the ultrasonic transducer elements of the ultrasonic transducer array (152) may be formed on the same chip as the electronics of the TX circuitry (154) and/or RX circuitry (156). The ultrasonic transducer arrays (152), TX circuitry (154), and RX circuitry (156) may be, in some embodiments, integrated in a single ultrasound probe. In some embodiments, the single ultrasound probe may be a hand-held probe including, but not limited to, the hand-held probes described below with reference to FIG. 9A. In other embodiments, the single ultrasound probe may be embodied in a patch that may be coupled to a patient. FIG. 9B provides a non-limiting illustration of such a patch. The patch may be configured to transmit, wirelessly, data collected by the patch to one or more external devices for further processing. In other embodiments, the single ultrasound probe may be embodied in a pill that may be swallowed by a patient. The pill may be configured to transmit, wirelessly, data collected by the ultrasound probe within the pill to one or more external devices for further processing.

A CMUT may include, for example, a cavity formed in a CMOS wafer, with a membrane overlying the cavity, and in some embodiments sealing the cavity. Electrodes may be provided to create an ultrasonic transducer cell from the covered cavity structure. The CMOS wafer may include integrated circuitry to which the ultrasonic transducer cell may be connected. The ultrasonic transducer cell and CMOS wafer may be monolithically integrated, thus forming an integrated ultrasonic transducer cell and integrated circuit on a single substrate (the CMOS wafer).

In the example shown, one or more output ports (164) may output a high-speed serial data stream generated by one or more components of the signal conditioning/processing circuit (160). Such data streams may be, for example, generated by one or more USB 3.0 modules, and/or one or more 10GB, 40GB, or 100GB Ethernet modules, integrated on the die (162). It is appreciated that other communication protocols may be used for the output ports (164).

In some embodiments, the signal stream produced on output port (164) can be provided to a computer, tablet, or smartphone for the generation and/or display of two-dimensional, three-dimensional, and/or tomographic images. In some embodiments, the signal provided at the output port (164) may be ultrasound data provided by the one or more beamformer components or auto-correlation approximation circuitry, where the ultrasound data may be used by the computer (external to the ultrasound device) for displaying the ultrasound images. In embodiments in which image formation capabilities are incorporated in the signal conditioning/processing circuit (160), even relatively low-power devices, such as smartphones or tablets which have only a limited amount of processing power and memory available for application execution, can display images using only a serial data stream from the output port (164). As noted above, the use of on-chip analog-to-digital conversion and a high-speed serial data link to offload a digital data stream is one of the features that helps facilitate an "ultrasound on a chip" solution according to some embodiments of the technology described herein.

Devices (150) such as that shown in FIG. 1B may be used in various imaging and/or treatment (e.g., HIFU) applications, and the particular examples described herein should not be viewed as limiting. In one illustrative implementation, for example, an imaging device including an N × M planar or substantially planar array of CMUT elements may itself be used to acquire an ultrasound image of a subject (e.g., a person's abdomen) by energizing some or all of the elements in the ultrasonic transducer array(s) 152 (either together or individually) during one or more transmit phases, and receiving and processing signals generated by some or all of the elements in the ultrasonic transducer array(s) (152) during one or more receive phases, such that during each receive phase the CMUT elements sense acoustic signals reflected by the subject. In other implementations, some of the elements in the ultrasonic transducer array(s) (152) may be used only to transmit acoustic signals and other elements in the same ultrasonic transducer array(s) (152) may be simultaneously used only to receive acoustic signals. Moreover, in some implementations, a single imaging device may include a P × Q array of individual devices, or a P × Q array of individual N × M planar arrays of CMUT elements, which components can be operated in parallel, sequentially, or according to some other timing scheme so as to allow data to be accumulated from a larger number of CMUT elements than can be embodied in a single device (150) or on a single die (162).

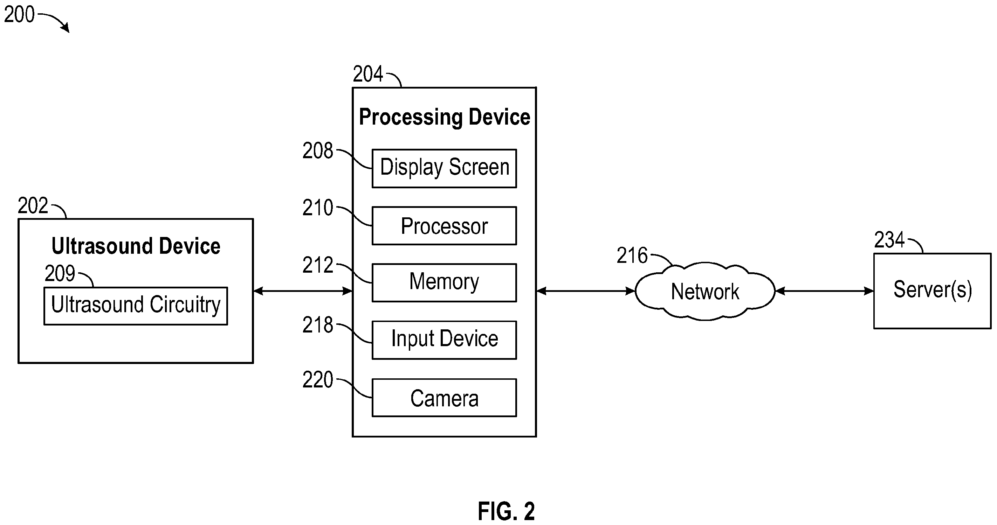

FIG. 2 illustrates a schematic block diagram of an example ultrasound system (200) which may implement various aspects of the technology described herein. In some embodiments, ultrasound system (200) may include an ultrasound device (202), an example of which is implemented in ultrasound device (150). For example, the ultrasound device (202) may be a handheld ultrasound probe. Additionally, the ultrasound system (200) may include a processing device (204), a communication network (216), and one or more servers (234). The ultrasound device (202) may be configured to generate ultrasound data that may be employed to generate an ultrasound image. The ultrasound device (202) may be constructed in any of a variety of ways. In some embodiments, the ultrasound device (202) includes a transmitter that transmits a signal to a transmit beamformer which in turn drives transducer elements within a transducer array to emit pulsed ultrasound signals into a structure, such as a patient. The pulsed ultrasound signals may be backscattered from structures in the body, such as blood cells or muscular tissue, to produce echoes that return to the transducer elements. These echoes may then be converted into electrical signals by the transducer elements and the electrical signals are received by a receiver. The electrical signals representing the received echoes are sent to a receive beam former that outputs ultrasound data. In some embodiments, the ultrasound device (202) may include an ultrasound circuitry (209) that may be configured to generate the ultrasound data. For example, the ultrasound device (202) may include semiconductor die (162) for implementing the various techniques described in.

Reference is now made to the processing device (204). In some embodiments, the processing device (204) may be communicatively coupled to the ultrasound device (202) (e.g., (102) in FIG. 1A) wirelessly or in a wired fashion (e.g., by a detachable cord or cable) to implement at least a portion of the process for approximating the auto-correlation of ultrasound signals. For example, one or more beamformer components may be implemented on the processing device (204). In some embodiments, the processing device (204) may include one or more processing devices (processors) (210), which may include specially programmed and/or special-purpose hardware such as an ASIC chip. The processor (210) may include one or more graphics processing units (GPUs) and/or one or more tensor processing units (TPUs). TPUs may be ASICs specifically designed for machine learning (e.g., deep learning). The TPUs may be employed to, for example, accelerate the inference phase of a neural network.

In some embodiments, the processing device (204) may be configured to process the ultrasound data received from the ultrasound device (202) to generate ultrasound images for display on the display screen (208). The processing may be performed by, for example, the processor(s) (210). The processor(s) (210) may also be adapted to control the acquisition of ultrasound data with the ultrasound device (202). The ultrasound data may be processed in real-time during a scanning session as the echo signals are received. In some embodiments, the displayed ultrasound image may be updated a rate of at least 5 Hz, at least 10 Hz, at least 20Hz, at a rate between 5 and 60 Hz, at a rate of more than 20 Hz. For example, ultrasound data may be acquired even as images are being generated based on previously acquired data and while a live ultrasound image is being displayed. As additional ultrasound data is acquired, additional frames or images generated from more-recently acquired ultrasound data are sequentially displayed. Additionally, or alternatively, the ultrasound data may be stored temporarily in a buffer during a scanning session and processed in less than real-time.

In some embodiments, the processing device (204) may be configured to perform various ultrasound operations using the processor(s) (210) (e.g., one or more computer hardware processors) and one or more articles of manufacture that include non-transitory computer-readable storage media such as the memory (212). The processor(s) (210) may control writing data to and reading data from the memory (212) in any suitable manner. To perform certain of the processes described herein, the processor(s) (210) may execute one or more processor executable instructions stored in one or more non-transitory computer-readable storage media (e.g., the memory (212)), which may serve as non-transitory computer-readable storage media storing processor-executable instructions for execution by the processor(s) (210).

The camera (220) may be configured to detect light (e.g., visible light) to form an image. The camera (220) may be on the same face of the processing device (204) as the display screen (208). The display screen (208) may be configured to display images and/or videos, and may be, for example, a liquid crystal display (LCD), a plasma display, and/or an organic light emitting diode (OLED) display on the processing device (204). The input device (218) may include one or more devices capable of receiving input from a user and transmitting the input to the processor(s) (210). For example, the input device (218) may include a keyboard, a mouse, a microphone, touch-enabled sensors on the display screen (208), and/or a microphone. The display screen (208), the input device (218), the camera (220), and/or other input/output interfaces (e.g., speaker) may be communicatively coupled to the processor(s) (210) and/or under the control of the processor (210).

It should be appreciated that the processing device (204) may be implemented in any of a variety of ways. For example, the processing device (204) may be implemented as a handheld device such as a mobile smartphone or a tablet. Thereby, a user of the ultrasound device (202) may be able to operate the ultrasound device (202) with one hand and hold the processing device (204) with another hand. In other examples, the processing device (204) may be implemented as a portable device that is not a handheld device, such as a laptop. In yet other examples, the processing device (204) may be implemented as a stationary device such as a desktop computer. The processing device (204) may be connected to the network (216) over a wired connection (e.g., via an Ethernet cable) and/or a wireless connection (e.g., over a WiFi network). The processing device (204) may thereby communicate with (e.g., transmit data to or receive data from) the one or more servers (234) over the network (216). For example, a party may provide from the server (234) to the processing device (204) processor-executable instructions for storing in one or more non- transitory computer-readable storage media (e.g., the memory (212)) which, when executed, may cause the processing device (204) to perform ultrasound processes. FIG. 2 should be understood to be non-limiting. For example, the ultrasound system (200) may include fewer or more components than shown and the processing device (204) and ultrasound device (202) may include fewer or more components than shown. In some embodiments, the processing device (204) may be part of the ultrasound device (202).

FIG. 2 illustrates an example handheld ultrasound probe, in accordance with certain embodiments described herein. The handheld ultrasound probe (280) may implement any of the ultrasound imaging devices described herein. The handheld ultrasound probe (280) may have a suitable dimension and weight. For example, the ultrasound probe (280) may have a cable for wired communication with a processing device, and have a length L about 100mm-300mm (e.g., 175 mm) and a weight about 200 grams-500 grams (e.g., 312 g). In another example, the ultrasound probe (280) may be capable of communicating with a processing device wirelessly. As such, the handheld ultrasound probe (280) may have a length about 140 mm and a weight about 265 g. It is appreciated that other dimensions and weight may be possible.

Further description of ultrasound devices and systems may be found in U.S. Patent No. 9,521,991 and U.S. Patent No. 11,311,274.

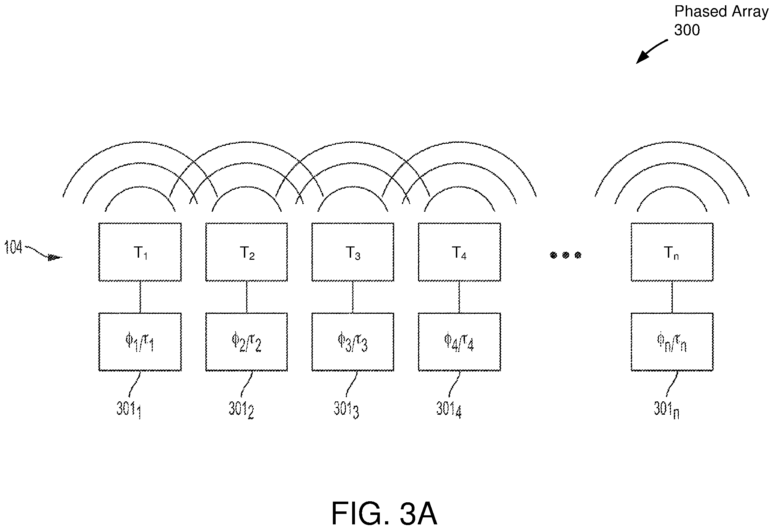

FIG. 3A shows a phased array (300), in accordance with one or more embodiments. In electrical or electronic steering, the ultrasound beams produced by different transducers in the array (152) may be oriented by adjusting the phases of the signals with which each transducer is driven. As such, the transducers may be arranged to form a phased array (300). In this configuration, an ultrasound device (150) comprises a plurality of ultrasound transducers T₁, T₂, T₃, T₄ ... T_{N}, where N may be greater than 10, greater than 100, greater than 1000, greater than 10000, or greater than 100000. The ultrasound transducers may be arranged as a two-dimensional array, as a one-dimensional array, or may be sparsely arranged. Each ultrasound transducers may be configured to receive a drive signal having a certain phase and a certain time delay. For example, ultrasound transducer T₁ is driven by a signal having a phase Φ₁ and a delay τ₁, ultrasound transducer T₂ is driven by a signal having a phase Φ₂ and a delay τ₂, ultrasound transducer T₃ is driven by a signal having a phase Φ₃ and a delay τ₃, ultrasound element T₄ is driven by a signal having a phase Φ₄ and a delay τ₄, and ultrasound transducer T_{N} is driven by a signal having a phase Φ_{N} and a delay τ_{N}. The phase and the delay of the drive signals may be controlled using signal drivers 301₁, 301₂, 301₃, 301₄, and 301_{N} (e.g., the TX circuitry (154) in FIGs. 1B and 2). The signal drivers may comprise phase shifters and/or adjustable time delay units. According to the manner in which the various phases are controlled relative to one another, the individual ultrasound waves emitted by the ultrasound elements may experience different degrees of interference (e.g., constructive interference, destructive interference, or any suitable value in between).

In some embodiments, the phases Φ₁, Φ₂, Φ₃, Φ₄ ... Φ_{N} and/or time delays τ₁, τ₂, τ₃, τ₄ ... τ_{N} may be controlled to cause the ultrasound waves to interfere with one another so that the resulting waves add together to increase the acoustic beam in a desired direction. The phases Φ₁, Φ₂, Φ₃, Φ₄ ... Φ_{N} and/or time delays τ₁, τ₂, τ₃, τ₄ ... τ_{N} may be controlled with respective signal drivers, which may be implemented for example using transistors and/or diodes arranged in a suitable configuration. In at least some of the embodiments in which the ultrasound elements are disposed on a semiconductor substrate, the signal drivers may be disposed on the same semiconductor substrate.

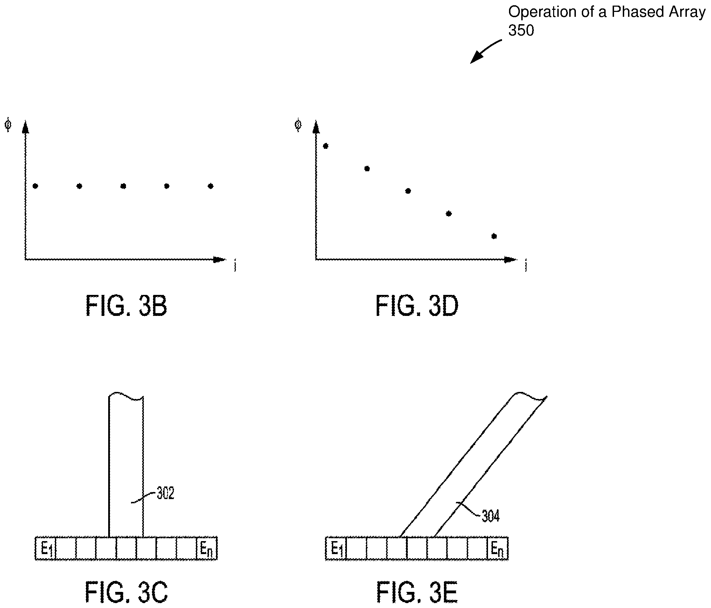

FIGs. 3B-3E show an operation (350) of a phased array in accordance with one or more embodiments. FIG. 3B is a plot illustrating the phase of the signal with which each transducer Tᵢ (i=1, 2 ... N) is driven. In the example, the ultrasound transducers are driven with uniform phases. As a result, the acoustic beam (302) is mainly directed along the perpendicular to the plane of the ultrasound device, as illustrated in FIG. 3C. In the example of FIG. 3D, the ultrasound transducers are driven with phases arranged according to a linear relationship. As a result, the acoustic beam (304) is angularly offset relative to the perpendicular to the plane of the ultrasound device, as illustrated in FIG. 3E. While not shown, the phases may be arranged in other manners. For example, the ultrasound transducers may be driven with phases arranged according to a quadratic relationship, which may result in an acoustic beam that converges. Any other suitable phase relationship may be used.

In some embodiments, the phases may be adjusted to produce steering within a 3D field-of-view. This may be accomplished for example by adjusting azimuth and elevation of the emissions. Accordingly, the acoustic beam may be steered through an entire volume at a desired angle, with respect to the direction that is normal to the aperture of the transducer array. While the above discussion is for the transmit phase, similar methods may be applied during the receive phase. Accordingly, beamforming in accordance with embodiments of the invention may involve beamforming when transmitting, when receiving, or both.

While not shown in FIGs. 3A-3E. the phased array may also be used for aperture translation. The concept of virtual aperture translation may be used to translate the aperture. An ultrasound device may transmit ultrasound pulses towards a set of focal points, and receive ultrasound pulses along a set of scan lines. These focal points may lie on a circle centered at a spatial point above the skin line known as the virtual apex. The angular extent of these focal points around the virtual apex is known as the image field of view (FOV) angle. Each transmitted pulse may be generated by a sub-set of the transducer array centered on the intersection of the line between the focal point and the virtual apex, except where this intersection falls beyond the transducer array, in which case the nearest sub-set of the transducer array is used. The scan lines may lie along a set of directions intersecting at the virtual apex. Aspects of the virtual apex are described in U.S. Patent Application No. 16/739,517 titled "METHODS AND APPARATUSES FOR ULTRASOUND IMAGING OF LUNGS" filed on January 10, 2020 (and assigned to the assignee of the instant application), published as U.S. Pat. Pub. No. 2020/01551 13 Al and issued as U.S. Pat. No. 10,709,415. The virtual apex may be shifted in the azimuthal direction and/or in the elevational direction. The virtual apex may be shifted between discrete positions, e.g., using 3 positions such as -.75cm, 0 cm, and 0.75 cm (0 cm being defined at the azimuthal center of the probe). Other numbers of positions and other sizes of the steps may be used without departing from the disclosure. The virtual apex may be shifted for transmit and/or receive beamforming. In one or more embodiments, the virtual apex is the same for both transmit and receive beamforming. It should be appreciated that because the position of the virtual apex may define the position of the aperture, translating the virtual apex from a particular reference position may result in translating the position of the aperture. In the case of azimuthal translation, the reference point may be, for example, the 0 cm position described above.

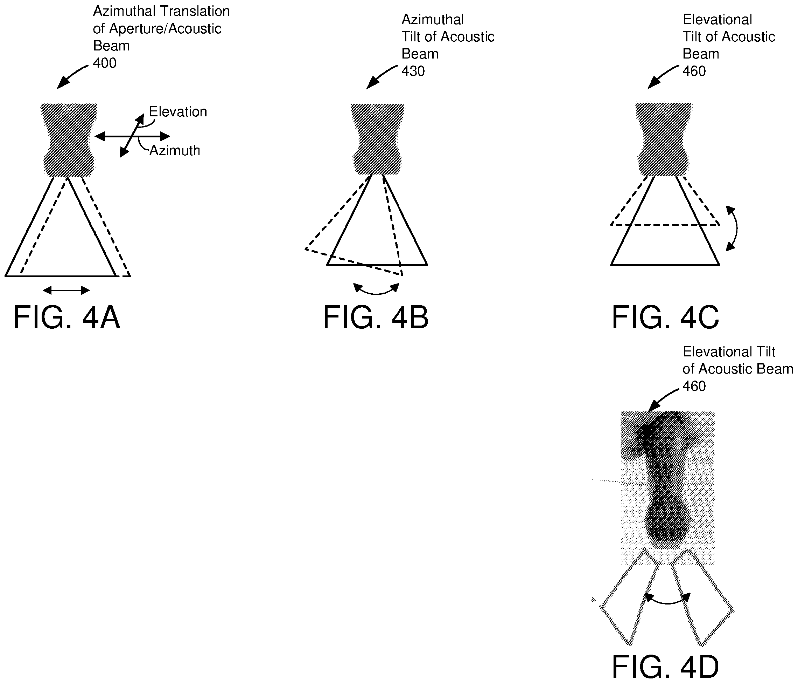

Turning to FIGs. 4A. 4B, 4C, and 4D examples of the application of acoustic beam steering to change the position and/or orientation of the imaging plane relative to the ultrasound probe, in accordance with one or more embodiments, are shown. As previously described, ultrasound probes in accordance with the disclosure (such as the ultrasound device (150)) have transducers organized in an array (152). Assuming that the array is two-dimensional, the steering described in reference to FIGs. 3A-3E may be performed in an azimuth direction and in an elevation direction. The steering may involve translation and/or tilt in the azimuth and/or elevation direction.

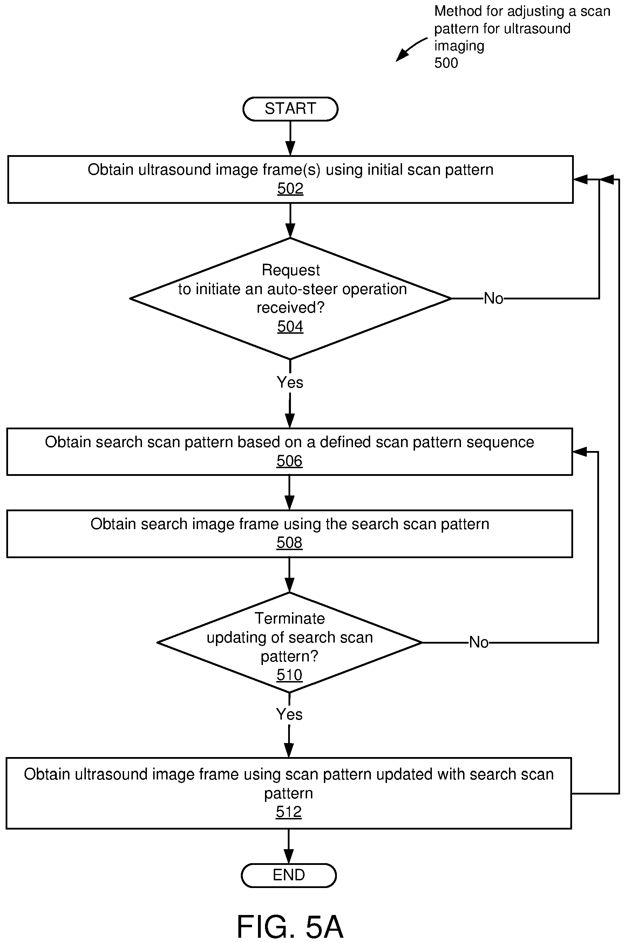

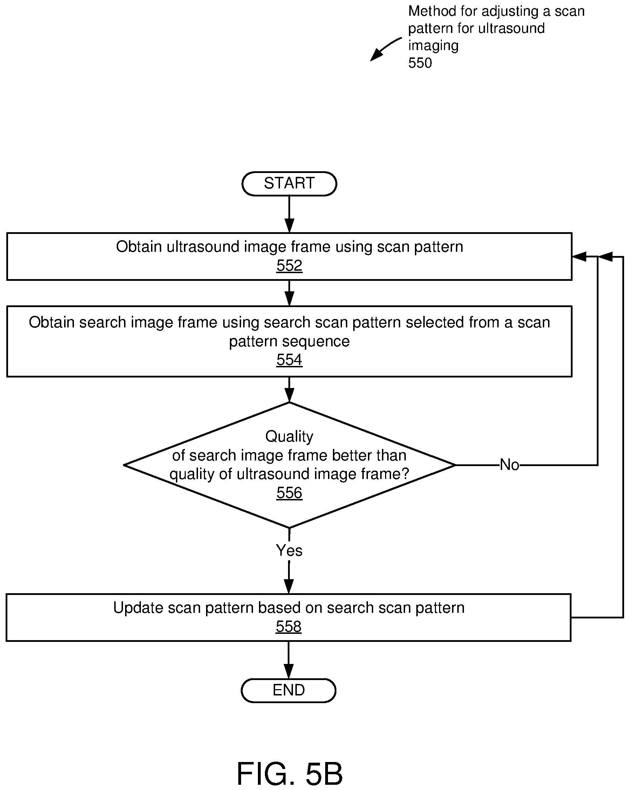

FIG. 4A shows an azimuthal aperture/acoustic beam (400) in accordance with one or more embodiments. As further discussed below, lung imaging is one application that may benefit from aperture translation. An optimization may be performed to determine a suitable azimuthal aperture position for the imaging. The optimization is described below in reference to FIGs. 5A and 5B. As described above, aperture translation may correspond to virtual apex translation from a particular reference point.

FIG. 4B shows an azimuthal tilt of the acoustic beam (430) in accordance with one or more embodiments. As further discussed below, cardiac imaging is an application that may benefit from the tilt. An optimization may be performed to determine a suitable azimuthal acoustic beam orientation for the imaging. The optimization is described below in reference to FIGs. 5A and 5B. The angle of an azimuthal tilt may be measured from a reference angle, for example, the angle normal to the transducer array.

FIGs. 4C and 4D show an elevational tilt of the acoustic beam (460) in accordance with one or more embodiments. The views of FIGs. 4C and 4D differ in that the ultrasound probe is rotated by 90°. As further discussed below, cardiac imaging and lung imaging are applications that may benefit from the tilt. An optimization may be performed to determine a suitable elevational orientation of the acoustic beam for the imaging. The optimization is described below in reference to FIGs. 5A and 5B. The angle of an elevational tilt may be measured from a reference angle, for example, the angle normal to the transducer array.

FIGs. 5A and 5B show flowcharts of methods in accordance with one or more embodiments. The methods may be performed to adjust a scan pattern for ultrasound imaging. More specifically, the methods may be performed to adjust the imaging plane to be used for an ultrasound imaging operation, as previously described in reference to FIGs. 4A-4D, followed by the use of the adjusted imaging plane to perform the ultrasound imaging. The imaging plane may be adjusted in different manners, depending on the application. For example, the adjustment of the image plane may be different depending on whether the ultrasound device is operating in a cardiac imaging mode or a lung imaging mode, as further discussed below. Broadly speaking, the methods of FIGs. 5A and 5B obtain a first ultrasound image frame using a previously established scan pattern. The first ultrasound image frame may include a view of a desired anatomy (e.g., a lung, a heart, etc.). The first ultrasound image may alternatively not include a view of the desired anatomy, e.g., if the ultrasound probe is improperly or poorly placed, or if the selected scan pattern is not suitable for capturing the desired anatomy. Next, the scan pattern is automatically updated to optimize the view of the desired anatomy. The automatic updating involves a processor, rather than a user, determining how to update the scan pattern. For example, the view may be optimized to avoid occlusions by other anatomical structures (e.g., bones). A second ultrasound image is then obtained using the updated scan pattern. The subsequently described steps may be performed in real-time, e.g., at a frame rate of an ultrasound system so that the imaging plane is adjusted in real-time. The subsequently described methods may be operated in different modes. For example, when in a cardiac imaging mode, the updating of the scan pattern may involve an adjustment of a combination of an azimuthal tilt and an elevational tilt (the azimuthal tilt and/or elevational tilt may be adjusted) of the acoustic beam (the azimuthal tilt and/or elevational tilt may be adjusted), and when in a lung imaging mode, the updating of the scan pattern may involve an adjustment of a combination of an elevational tilt and a translation of the aperture (the elevational tilt and/or the aperture translation may be adjusted) .

One or more steps shown in FIGs. 5A and 5B may be omitted, repeated, and/or performed in a different order than the order shown in FIGs, 5A and 5B. Accordingly, the scope of the invention should not be limited by the specific arrangement as depicted in FIGs. 5A and 5B. Additionally, according to one or more embodiments, the method depicted in FIGs. 5A and 5B (and described below) may be implemented using the above-described ultrasound system (100) as well as any variation of the ultrasound system (100) or any other suitable system or apparatus. Some or all of the steps in the method depicted in FIGs. 5A and 5B may be performed by a processor, and the instructions for performing these steps may be stored in a non-transitory computer readable memory.

Turning to FIG. 5A, in Step 502, after the ultrasound probe has been positioned by the user, an ultrasound image frame (or any number of ultrasound image frames) is obtained. The ultrasound probe may have been positioned by the user to obtain ultrasound image frames of a desired anatomy. The positioning of the ultrasound probe may have been performed to the best of the user's knowledge, i.e., the ultrasound probe may be suboptimally placed, particularly when the user is inexperienced. In one or more embodiments, the ultrasound image frame is captured using a particular scan pattern. The scan pattern may be a standard scan pattern of the ultrasound probe defined by a scan pattern, described below, may be a result of a prior scan pattern, and/or may have been specifically selected by the user. In one embodiment, the initial scan pattern includes an elevational angle=0 and azimuthal angle=0, i.e., perpendicular to the surface of the transducer array in both the elevational plane and the azimuthal plane. Other aspects of the scan pattern may be specified as well, e.g., an aperture position, aperture size, aperture orientation, etc. Those skilled in the art will recognize that configuring the ultrasound device to obtain an ultrasound image frame does not mean that the ultrasound device must necessarily produce a final ultrasound image frame. Rather, the ultrasound device may collect ultrasound data that may be used by another device, such as the smartphone or tablet, to produce the final ultrasound image frame.

In Step 504, a test is performed to determine whether a request is received to initiate an auto-steer operation. The request may be based on a user request, or it may be an ultrasound device-internal request. The user request may be the pressing of a button, e.g., on the ultrasound probe, by the user. The ultrasound device-internal request, in one or more embodiments is based on a suitability of an ultrasound image frame for performing a diagnostic action. Quality-based metrics or other metrics may be used. The metrics may be continuously or periodically monitored in order to make the suitability determination. Specifically, for example, if the quality of the ultrasound image frame falls below a threshold, then this may trigger the auto-steer operation. In one or more embodiments, quality is at least partially governed by the view of the desired anatomy. For example, a lower quality may result from significant occlusions of parts of the desired anatomy. Higher quality may result from fewer or no occlusions of the desired anatomy. A discussion of quality is provided below, following the description of the methods. Step 504 is optional. In other words, in some embodiments, the subsequently described steps may be executed without being explicitly triggered by Step 504.

If no request was received, the method may proceed with another execution of Step 502. Accordingly, another ultrasound image frame may be obtained using the initial scan pattern.

If a request was received, a search mode or auto-steer operation is activated. Briefly summarized, the auto-steer operation, performed by executing subsequently described Steps 506-510, adjusts the scan pattern to be used to capture the ultrasound images, with the goal to identify a scan pattern that provides better quality ultrasound image frames (or more broadly, ultrasound images with a higher suitability for the purpose of performing a diagnostic action). For example, in one embodiment in which a scan pattern sequence defines a number of scan patterns, e.g., elevational and azimuthal steering angles for the acoustic beam, execution of Steps 506-510 may result in variation of the elevational and azimuthal steering angles. An updated scan pattern based on a combination of elevational and azimuthal steering angles may then be used to capture ultrasound image frames after completion of the auto-steer operations. Examples and a description of sequences of scan patterns are provided below, following the description of the methods. The user may be instructed to hold the ultrasound probe still while different steering angles are explored while the auto-steer operations are ongoing.

In Step 506, triggered by the request, a search scan pattern is determined based on one or more of the subsequently discussed sequences of scan patterns. Specifically, for example, the search scan pattern may be set to include exploratory steering angles and/or positions for the acoustic beam, specified in a scan pattern sequence. A scan pattern sequence may include many definitions of scan patterns in the form of combinations of steering angles and/or positions of the aperture, and by repeatedly executing Steps 506-510, the method may step through some or all of these combinations.

In Step 508, a search image frame is obtained. The search scan pattern is used to capture the search image frame. The ultrasound probe may have remained stationary or near-stationary between the execution of Steps 502 and 508.

In Step 510, a decision is made whether the auto-steer operation should be terminated. Different criteria may be used to make the decision. For example, the auto-steer operation may be terminated once a predetermined sequence of exploratory steering angles, specified in a scan pattern sequence, has been used to obtain search image frames with the corresponding search scan pattern. Additionally or alternatively, the auto-steer operation may be terminated once a search image frame obtained using a particular search scan pattern provides sufficient image quality, e.g., meeting specified quality standards or being higher quality than the ultrasound image frame obtained in Step 502. More generally, the auto-steer operation may be determined when a search image frame is deemed sufficiently suitable for performing a diagnostic action.

If the updating of the search scan pattern is to be continued, the execution of the method may proceed with Step 506 to pick another scan pattern from the scan pattern sequence. If the updating of the search scan pattern is to be terminated, the method may proceed with Step 512.

In Step 512, an ultrasound image frame (or any number of ultrasound image frames) is obtained. An updated scan pattern is used for capturing the ultrasound image frame. The updated scan pattern may be set based on the search scan pattern that produced a highest suitability for the purpose of performing a diagnostic action, a highest quality, a quality above a threshold, etc., during the execution of Steps 506-510. The execution of Step 512 may continue for a prolonged time, for example, until the user decides that sufficient number of ultrasound image frames has been collected. Eventually, the execution may stop, or alternatively, the execution of the method may repeat, for example, after the ultrasound probe has been moved to a different location.

Turning to FIG. 5B. in Step 552, after the ultrasound probe has been positioned by the user, an ultrasound image frame (or any number of ultrasound image frames) is obtained. The ultrasound probe may have been positioned by the user to obtain ultrasound image frames of a desired anatomy. The positioning of the ultrasound probe may have been performed to the best of the user's knowledge, i.e., the ultrasound probe may be suboptimally placed, particularly when the user is inexperienced. In one or more embodiments, the ultrasound image frame is captured using a scan pattern that is repeatedly updated as the method of FIG. 5B is executed in iterations. Initially, the scan pattern may be a standard scan pattern of the ultrasound probe defined by a scan pattern, described below, may be a result of a prior scan pattern, and/or may have been specifically selected by the user. In one embodiment, an initial scan pattern is used during the initial execution of the method of FIG. 5B. In one embodiment, the initial scan pattern includes an elevational angle=0 and azimuthal angle=0, i.e.. perpendicular to the surface of the transducer array in both the elevational plane and the azimuthal plane. Those skilled in the art will recognize that configuring the ultrasound device to obtain an ultrasound image frame does not mean that the ultrasound device must necessarily produce a final ultrasound image frame. Rather, the ultrasound device may collect ultrasound data that may be used by another device, such as the smartphone or tablet, to produce the final ultrasound image frame.

The following steps for an auto-steer operation may be performed based on a request, or without being explicitly triggered by a request. The request may be based on a user request, or it may be an ultrasound device-internal request. The user request may be the pressing of a button, e.g., on the ultrasound probe, by the user. The ultrasound device-internal request, in one or more embodiments is suitability-based. In other words, if the suitability of the ultrasound image frame for the purpose of performing a diagnostic action is too low, an auto-steer operation may be triggered. Quality-based metrics or other metrics may be used. Specifically, for example, if the quality of the ultrasound image frame falls below a threshold, then this may trigger the auto-steer operation. In one or more embodiments, quality is at least partially governed by the view of the desired anatomy. For example, a lower quality may result from significant occlusions of parts of the desired anatomy. Higher quality may result from fewer or no occlusions of the desired anatomy. A discussion of quality is provided below, following the description of the methods.

Briefly summarized, the auto-steer operation, performed by executing subsequently described Steps 554-558 adjusts the scan pattern to be used to capture the ultrasound images, with the goal to identify a scan pattern that provides better quality ultrasound image frames (or, more generally, a higher suitability of the ultrasound image frames for the purpose of performing a diagnostic action). In the embodiment as shown, execution of Steps 554-558 may result in an incremental updating of the scan pattern by interleaving search image frames between the ultrasound image frames. The user may be instructed to hold the ultrasound probe still while different steering angles are explored during the auto-steer operations.

For example, the execution of the method may begin with an imaging at elevational angle=0 and an azimuthal angle=0, but with search image frames added between the ultrasound image frames, the search image frames with a different angle every N (e.g., 3 or 4) frames, so as not to degrade the frame rate too much. The search image frames may be hidden from the user (i.e., they may not be displayed), but their quality may be computed. If a search image frame produces a better quality than the previously obtained ultrasound imaging frame, one of two things may occur. Either the user may be notified (visually, audibly, or otherwise) that a more suitable scan pattern was identified and asked whether they would like to switch to that scan pattern, or the ultrasound device may automatically update the scan pattern to produce better quality ultrasound image frames. Searching may continue, interleaving search frames at other angles. These operations are performed by repetitive execution of Steps 552-558.

In Step 554, triggered by the request, a search image frame is obtained using a search scan pattern. The search scan pattern may be determined by stepping through the scan pattern sequence, that define exploratory steering angles and/or positions for the acoustic beam, as previously discussed. Based on the selected search scan pattern, a search image frame is obtained. By repeated execution of Step 556 in a loop, many combinations of steering angles and/or positions of the aperture may be used to obtain search image frames.

In Step 556, a test is performed to determine whether the quality of the search image frame is better than the quality of the previously obtained ultrasound image frame. A comparison may be performed based on quality metrics, as further discussed below. If the quality of the search image is not better, then the execution of the method may proceed with Step 552 to repeat obtaining an ultrasound image frame, without updating the scan pattern, followed by producing a different search image frame using a different search scan pattern. If the quality of the search image frame is better, then the execution of the method may proceed with Step 558.

In Step 558, the scan pattern is updated based on the search scan pattern that resulted in the search image frame with the quality better than the quality of the ultrasound image frame. For example, the configuration for the initial scan pattern is replaced with the configuration of the search scan pattern. Subsequently, when returning to Step 552, a new ultrasound image may be obtained using the updated search scan pattern.

### Scan Patterns

Scan patterns establish possible combinations of scan parameters to be used for beam steering when executing the methods (500, 550). The following description provides definitions of scan patterns.
- A scan pattern may involve definitions for an aperture/acoustic beam translation in elevation and/or azimuth directions, e.g., as illustrated for an azimuthal translation in FIG. 4A. As described above, translating the aperture may correspond to translating the virtual apex. A particular aperture translation may be measured from a reference position as described above.
- A scan pattern may involve definitions for an acoustic beam tilting in azimuth, e.g., as illustrated in FIG. 4B. As described above, tilting the acoustic beam in azimuth may be measured from a reference angle.
- A scan pattern may involve definitions for an acoustic beam tilting in elevation, e.g., as illustrated in FIGs. 4C and 4D. As described above, tilting the acoustic beam in elevation may be measured from a reference angle.
- A scan pattern may involve definitions for an aperture/acoustic beam rotation about the probe axis (i.e., about an axis orthogonal to the aperture) through coupled azimuth and elevation steering.

Any combination of the above definitions (and/or any definitions of translations, tilts, rotations, etc. of the acoustic beam) may be arranged in a sequence to form a scan pattern sequence, as further discussed below.

Non-limiting examples of such image frame sequences are shown below in Tables 1 and 2. These tables present mini-sequences of imaging frames with interleaved search frames, e.g.. as used by the method (550).

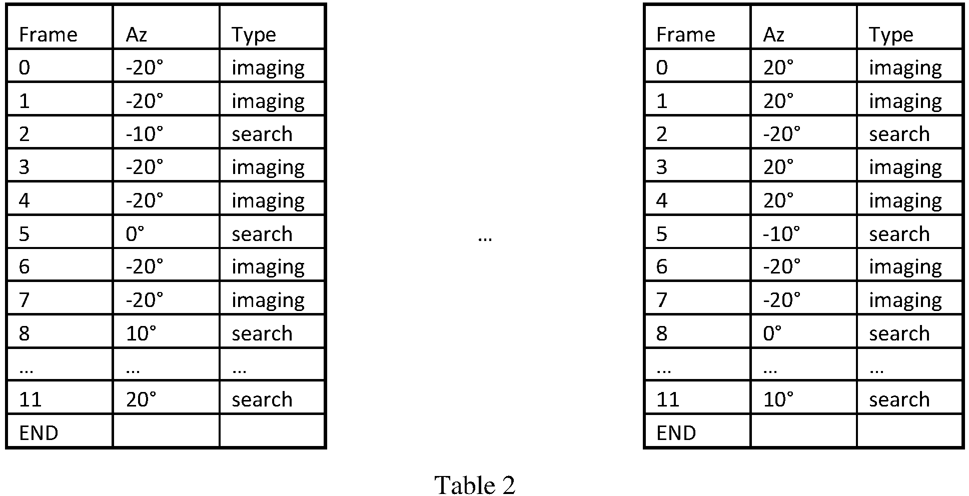

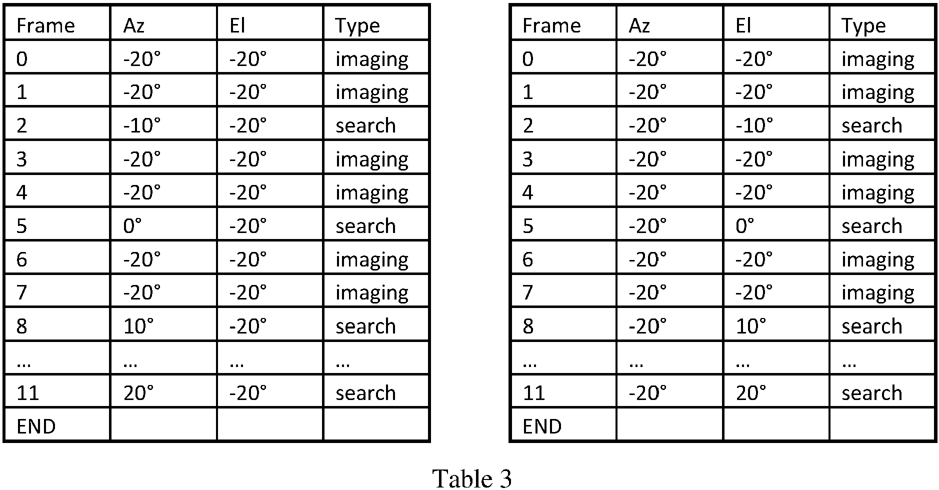

The scan pattern sequence in Table 2 differs from that in Table 3 in that the sequence of Table 2 adjust the azimuthal angle only, while the sequence in Table 3 adjust both azimuth and elevation angles. The results of the scan sequences illustrated in Tables 2 and 3 may be used to build a multidimensional numerical optimizer. Such an optimizer may be used to better find an optimal imaging angle.

### Stepping Through a Scan Pattern Sequence

The stepping through a scan pattern sequence when performing an auto-steer operation as described in reference to FIGs. 5A and 5B, may be performed in various different manners:
- In one embodiment, a full sweep is performed. The full sweep may involve all scan patterns. For example, for scan pattern sequences that vary elevational and an azimuthal tilt, this would mean stepping through all combinations of elevation and azimuth, either until all combinations have been tested or until a specified quality of the search image is achieved. Optionally, in one embodiment, the full sweep may be performed using a coarse sweep, followed by a fine sweep. For instance, the coarse sweep may identify a suitable range and a fine sweep may subsequently identify a desired scan pattern within the range identified by the coarse sweep.
- In one embodiment, a decoupled elevational and azimuthal search may be performed, e.g., by sweeping over an elevation at the current azimuth to update the elevation angle. Next, a sweep over the azimuth at the current elevation may be performed to update the azimuthal angle. This may be repeated. The approach may identify the target view without needing to test all potential combinations of elevation steering angle and azimuth steering angle, leading to quicker identification of the most appropriate steering angles and lower power consumption. Optionally, several planes (or patterns) may be simultaneously scanned, when iterating towards the best view (e.g., larger elevation).
- In one embodiment, methods of artificial intelligence (AI) are used to infer how to adjust the scan pattern to yield the desired scan pattern. For instance, using AI, the precise adjustment to the steering angle(s) may be identified from information in the current ultrasound image without needing to test alternative steering angles, or as many steering angles. An AI model may be built, for example, by training the AI model on ultrasound image frames each manually labeled with the appropriate change in scan pattern to acquire a better image. Through the training, the model learns to predict the change in scan pattern for any given ultrasound image frame.

While the described stepping through scan patterns in a scan pattern sequence is based on examples involving azimuth and elevation, this may be generalized to any type of scan patterns that may include elevation and/or azimuth angle changes, translations in elevation and/or azimuth directions, rotations about the ultrasound probe axis, etc.

### Assessing Suitability of an Ultrasound Image Frame / Search Image Frame

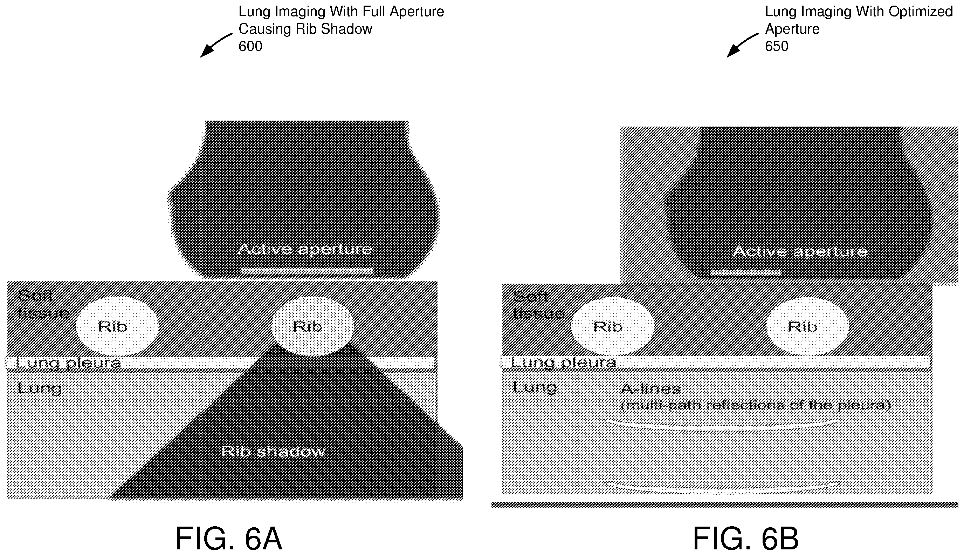

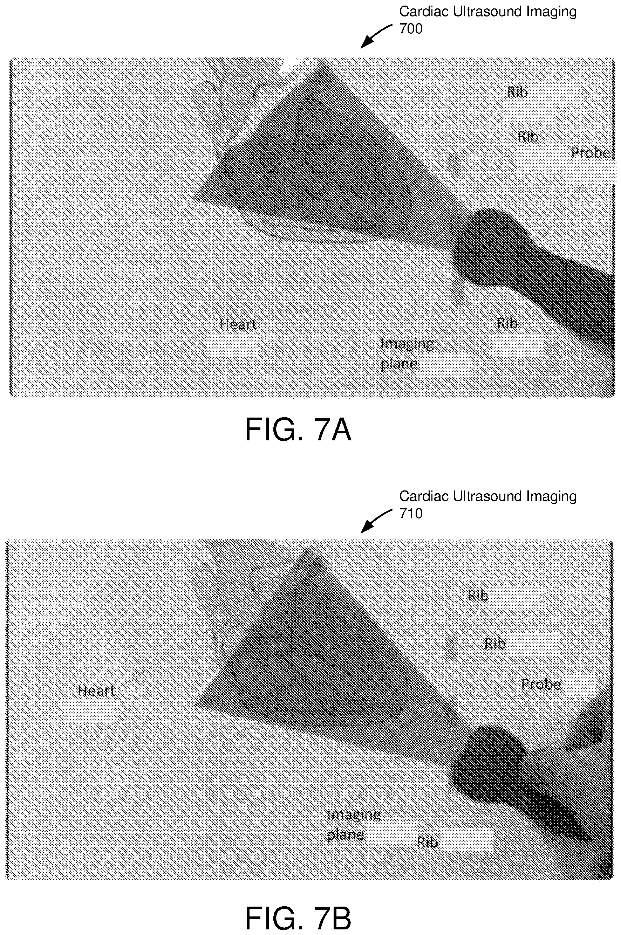

In one or more embodiments, one or more ultrasound image frame/search image frame is scored to decide on suitability. Suitability may be governed by the diagnostic action being performed. In other words, a suitable ultrasound image frame/search image frame is likely or sufficiently likely to be useful for the diagnostic action, whereas a non-suitable ultrasound image frame/search image frame is not sufficiently likely to be useful for the diagnostic action. In one or more embodiments, suitability is at least partially governed by the view of the desired anatomy in the ultrasound image frame / search image frame. For example, a lower suitability may result from significant occlusions of parts of the desired anatomy. Higher suitability may result from fewer or no occlusions of the desired anatomy. A threshold may be applied to distinguish good/acceptable suitability and bad suitability. Examples for specific applications are provided below in reference to FIGs. 6A and 6B, and FIGs. 7A-7C.

In one embodiment, a quality of the ultrasound image frame / search image frame is evaluated to assess suitability. Various methods may be used to assess quality:
- Artificial intelligence (AI) may be used to determine whether am image frame is appropriate for diagnostic use. For example, the types of quality indicators and manner of determining quality indicators described in U.S. Pat. 10,628,932 (the '932 patent may be employed. Alternatively, or in addition, the types of quality indicators and manner of determining quality indicators described in U.S. Pat. Pub. 2020/0372657 AI (the '657 publication) may be employed. The '932 patent and the '657 publication are co-owned with the current application. One example for determining quality involves an assessment of the image frame to estimate a probability that a medical professional would rely on the ultrasound imaging for clinical use. Threshold probabilities may be used to make such a decision. The AI algorithm making the determination may have been trained using historical data including image frames and labels indicating their quality. The labeling may have been performed by an individual or by groups of individuals to obtain collective evaluations of the quality.
- AI may be used to determine whether an image frame contains the greatest amount of the desired anatomy. Methods of image processing may be used to make this determination.
- Non-AI approaches may be used. Non-AI approaches may rely on relatively simple image metrics. For example, operations may be performed to identify a maximum degree of motion in a set of image frames captured over time, e.g., when working with cardiac images.
- Various possible extensions of the methods, described in reference to FIGs. 5A and 5B, are subsequently discussed.

The above description assumes that the ultrasound probe is kept stationary or near-stationary during the execution of the method steps. However, even with the described capability to adjust the scan pattern to obtain an acceptable ultrasound image frame with the desired anatomy without user intervention, there may be cases where the ultrasound probe must be physically moved on the body to acquire the desired view, In a "cardiac" mode, for instance, the user might position the probe near the heart, then attempt to scan for the desired view. If the desired view is not accessible from the current probe position, the system, in one embodiment, alerts the user to "move the probe towards the patient's head" via text instructions and/or graphical depictions. The system may then continue automatic scanning for the desired view, and after several probe movements, the desired view may be accessible with an appropriate scan pattern.

Further, embodiments of the disclosure address concerns regarding lung interference. For example, when operating in a cardiac scanning mode, the system may detect lung interference and alert the user to instruct the patient to breathe out and hold, which may move the lung out of the beam path.

Also, embodiments of the disclosure provide guidance to the user to perform more complex imaging operations. For example, in FAST (focused assessment with sonography in trauma) imaging, an ultrasound study that includes views of the heart, lungs, Morrison's pouch, etc. is conducted to searching for free fluid indicating internal bleeding. In one or more embodiments, the ultrasound device instructs the user to move the probe to the patient's right upper quadrant, followed by an automatic adjustment of the scan pattern to search for free fluid. Then, instructions may be provided to move the probe to near the patient's heart, followed by an automatic adjustment of the scan pattern to search for free fluid, etc. In this scenario, the scan pattern may be adjusted to search for free fluid in 3-dimensional space via elevational sweeping, instead of merely trying to identify a single view of the desired anatomy.

Embodiments of the disclosure may be implemented in different configurations. As previously described, a host device (e.g., a smartphone or a tablet computing device) may be communicating with an AI model in the cloud. Alternatively, the AI model would be run locally on the ultrasound probe in an Edge AI configuration. In such a configuration, the algorithms may utilize the data generated by the devices themselves, and the devices may make independent decisions in a matter of milliseconds without having to connect to the Internet/cloud.

One or more embodiments of the disclosure support fast sequence changes.

Adjusting the scan pattern may require full re-computation of sensor delays and gains, as well as beamforming parameters used to reconstruct data along the new plane. This operation may require a non-negligible amount of time, e.g. 100-500ms. Such a delay may introduce a lag while scanning. This lag may be undesirable or even unacceptable, especially if the search frames are interleaved with the imaging frames. To avoid this lag, ultrasound devices in accordance with embodiments of the disclosure may compute the new imaging parameters with a different processor than the one currently used for imaging. Alternatively, ultrasound devices in accordance with embodiments of the disclosure may pre-generate a "complete" sequence that contains many different scan patterns, allowing the host to quickly switch between them by pointing the system to different points in memory.

The following paragraphs illustrate non-limiting examples of applications of embodiments of the disclosure. Lung imaging and cardiac imaging are discussed. As the examples illustrate, in lung imaging, elevational steering may ensure that the imaging plane is perpendicular to the lung pleura, whereas in cardiac imaging, it may be used to get a better window through the ribs. Accordingly, while both cardiac and lung imaging use elevational autosteering, it is for different purposes.

FIGs. 6A and 6B show an ultrasound imaging of a lung in accordance with one or more embodiments. As subsequently discussed, in lung imaging, the azimuthal acoustic beam is oriented perpendicular to the direction of the ribs. An aperture/acoustic beam translation may be used to avoid rib shadowing that occurs when the center of the aperture is directly above a rib. In this situation, tilting the acoustic beam would not help because the rib would still partially block the acoustic beam. However, translating the aperture may move the acoustic beam to a position where the center of the acoustic beam is between the ribs rather than on top of them.

FIG. 6A illustrates an attempt to image a lung (600) using a wide active aperture, thereby causing a prominent rib shadow. The rib shadow may be sufficiently prominent to prevent a meaningful interpretation of the resulting ultrasound image. FIG. 6B illustrates a lung imaging using an optimized scan pattern (650) when the ultrasound device is operating in a lung imaging mode, in accordance with one or more embodiments. While, the position of the ultrasound probe is the same in FIGs. 6A and 6B, in FIG. 6B the active aperture is optimized in order to reduce the rib shadow. More specifically, the narrower active aperture is optimized in the azimuth translational direction to reduce the effect of the rib under the ultrasound probe. Further, while not shown in the illustration of FIG. 6B, an elevational steering is further performed. The elevational steering may ensure that the imaging plane, i.e., the elevation plane, is perpendicular to the lung pleura. Assuming, for example, that the ultrasound probe was tilted forward or backward, then the imaging plane would traverse the tissues (e.g., soft tissue, lung pleura) at an oblique angle. By performing an elevational tilting to correct for the tilted ultrasound probe, it can be ensured that the elevational plane is hitting the tissue at a 90 degree angle. This may be of relevance, for example, when trying to obtain the A-lines shown in FIG. 6B, i.e., repetitive horizontal echoic lines that arise from the lung pleura at regular intervals. These A lines are a result of total reflection of the ultrasound beam at the air-tissue interface formed by the lung pleura in an intact lung.

FIGs. 7A-7C show an ultrasound imaging of a heart in accordance with one or more embodiments. In cardiac scanning, the ultrasound probe may be oriented to be parallel with the direction of the ribs, allowing tilting and steer in both (azimuth and elevation) directions in order to get a better angle for viewing the heart.

As explained above, aspects of the present application provide for automatically adjusting the scan pattern of an ultrasound imaging device, such as an ultrasound probe or patch, to obtain a desired or target view of the specified anatomy without user intervention, with such operation being beneficial in the context of cardiac imaging. While the technology described herein may be applied to imaging of various anatomical features, cardiac imaging presents particular challenges because the "cardiac windows" through the ribs and lungs can be hard to find, and may vary from patient to patient. Fine probe movements are typically required to find the correct view with conventional ultrasound probes, and thus novices often struggle to obtain acceptable cardiac images. That is, successful cardiac imaging often involves making fine adjustments in the position and angle of the ultrasound probe, and a suitable position and angle often depends on the anatomy, position, and breath state of the particular subject being imaged. Thus, application of the present technology is particularly beneficial for at least some cardiac imaging scenarios. FIGs. 7A and 7B illustrate a sequence of probe positions relative to a subject as part of a cardiac imaging session. The illustrated probe positions exhibit different benefits and drawbacks.

FIG. 7A illustrates an ultrasound imaging (700) of the heart. The ultrasound probe is positioned between two ribs, such that the imaging plane is directed generally toward the heart. In this position, the probe is stable, because of it being wedged between the two ribs. However, the imaging plane is significantly obstructed by one of the ribs. Thus, the resulting image would show significant haze/clutter, and the overall quality of imaging would be poor. This is particularly true when the probe head is larger than the space between the two ribs, as is the case for some portable ultrasound probes, such as those designed to operate at higher frequencies for which it is beneficial to have a larger probe face.

FIG. 7B illustrates an ultrasound imaging (710) of the heart. In comparison to FIG. 7A. the ultrasound probe is moved to a new, lower position in which the probe head is positioned mechanically against one of the ribs. In this position, the imaging plane is less obstructed by the ribs, and the quality of the resulting image would be better than in the position of FIG. 7A. However, placement of the probe head against the rib such that a small portion such as a point-of the probe makes contact with the subject is unstable, such that the probe may easily and inadvertently be tilted and or translated. Such unstable positioning results in low quality imaging and can in some situations entirely ruin the view of the heart.

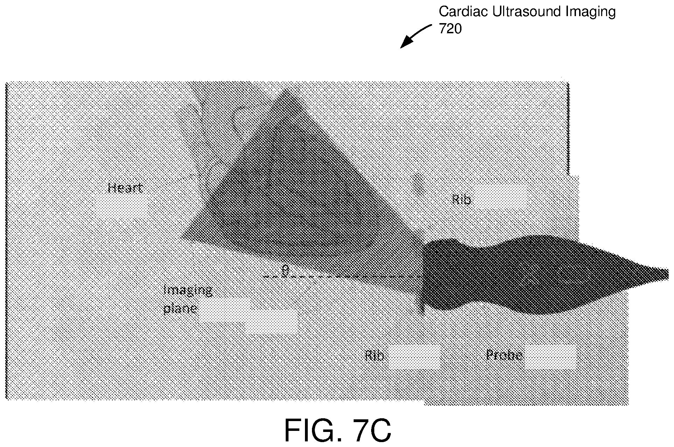

FIG. 7C illustrates an ultrasound imaging (720) of the heart when the ultrasound device is operating in a cardiac imaging mode, in accordance with one or more embodiments of the disclosure, in which automatic steering of the imaging plane is used. As shown in FIG. 7C, the ultrasound probe is positioned in a stable manner against two ribs, with the probe positioned flat against the chest. In this position, the normal from the probe face does not intersect the heart. However, the imaging plane is angled at an angle θ relative to the normal, such that the imaging plane intersects the heart without significant obstruction from the ribs. The angle β may be determined automatically by the ultrasound imaging system in any of the manners described above. Thus, it should be appreciated that high quality cardiac imaging may be achieved with stable probe positioning using the techniques described herein, and that the conventional fine mechanical adjustments made by the operator may be unnecessary.

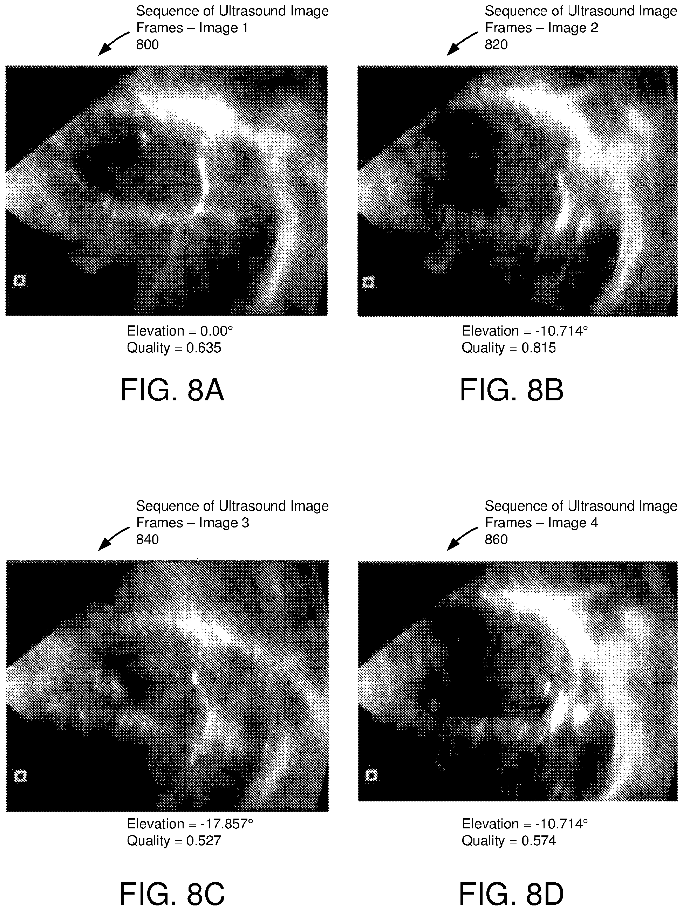

FIGs. 8A 8D illustrate a sequence of ultrasound image frames taken of a subject's heart, showing the quality factor associated with each image. As shown, the ultrasound system may select the elevation of the ultrasound beam to be the elevation associated with the highest quality for a given tilt of the ultrasound probe relative to the subject. FIG. 8A illustrates an ultrasound image collected at an elevational steering angle of 0.00 degrees. This elevational steering angle may be selected when the ultrasound probe is substantially perpendicular to the subject, as a non-limiting example. At this angle, the quality of the resulting image is calculated to be 0.635.

The probe is then tilted relative to the subject, such that the probe face is directed more downwardly toward the subject's feet. The system detects that, absent adjusting the elevational steering angle, the resulting image quality drops below a threshold and therefore performs a steering angle sweep to identify the steering angle producing the best image quality for the given probe tilt. FIG. 8B shows that the elevation of -10.714 degrees produces a quality of 0.815 which is determined to be the best quality for that particular probe tilt. Thus, it should be appreciated that in this figure, and more generally in the series of FIGs. 8A-8D, a negative elevation is selected when the probe face is tilted more toward the subject's feet. By contrast, a positive elevation is selected when the probe face is tilted more toward the subject's head. The probe is then tilted downward toward the subject's feet to a greater degree. The system detects that the resulting image quality drops below a threshold, which may be the same as the prior threshold or may be a different threshold in some embodiments. An elevation angle sweep is therefore performed, and as shown in FIG. 8C an elevation of ..17.857 degrees produces a quality of 0.527 which is determined to be the best quality for that particular probe tilt. The probe is then tilted upward (toward the subject's head) to a greater degree. The system detects that the resulting image quality drops below a threshold, which may be the same as a prior threshold or may be different, and therefore performs a steering angle sweep. FIG. 8D shows that the elevation of -10.714 degrees produces a quality of 0.574 which is determined to be the best quality for that particular probe tilt. Thus, the sequence of FIGs. 8A-8D illustrates how the quality of the ultrasound image may be impacted by the elevation angle of the ultrasound beam, and how the elevation angle may be automatically selected to correspond to an image quality above a threshold value.

Although FIGs. 8A-8D are directed to the specific setting of cardiac imaging, it should be appreciated that the automatic ultrasound imaging techniques described may be applied to imaging of other anatomic features as well.

FIG. 9A illustrates an example handheld ultrasound probe, in accordance with certain embodiments described herein. Handheld ultrasound probe (900) may implement the ultrasound device (150) in FIG. 1B. The handheld ultrasound probe (900) may correspond to the ultrasound device (102) in operative communication with the processing device (104) in FIG. 1A and may transmit the detected signals to the computer device. Alternatively and/or additionally, the ultrasound probe (900) may include an ultrasound device and a processing device for performing operations over ultrasound signals received from the ultrasonic transducer. In some embodiments, the handheld ultrasound probe (900) may be configured to communicate with the processing device (104) wired or wirelessly. The ultrasound probe (900) may have a cable for wired communication with a processing device, and have a length L about 100mm-300mm (e.g., 175 mm) and a weight about 200 grams-500 grams (e.g., 312 g). In another example, the ultrasound probe 1100 may be capable of communicating with a processing device wirelessly. As such, the handheld ultrasound probe 1100 may have a length about 140 mm and a weight about 265 g. It is appreciated that other dimensions and weights may be possible.

FIG. 9B illustrates an example wearable ultrasound patch, in accordance with certain embodiments described herein. The wearable ultrasound patch (950) is coupled to a subject (952). The wearable ultrasound patch (950) may implement some or all of the elements of the ultrasound device (150) of FIG. 1B.

More generally, the described operations, including the operations of the methods described in FIGs. 5A and 5B, may be performed on a computer device such as a smartphone or a tablet computer, on an ultrasound probe, on an ultrasound patch, or distributed between these components.

Although the disclosure has been described with respect to a limited number of embodiments, those skilled in the art, having benefit of this disclosure, will appreciate that various other embodiments may be devised without departing from the scope of the present disclosure. Accordingly, the scope of the disclosure should be limited only by the attached claims.

## Claims

1. An ultrasound system (100) comprising:
an ultrasound device (102) with a two-dimensional array (152) of ultrasound transducers, the ultrasound device implemented as a handheld device or a wearable patch; and
a smartphone or tablet comprising a processor (104) that
configures the ultrasound device to obtain at least one first ultrasound image frame, using a scan pattern defining an acoustic beam,
automatically updates the scan pattern to optimize a view of a desired anatomy, wherein:
when the ultrasound system is operating in a cardiac imaging mode, the updating of the scan pattern comprises an adjustment of a combination of an azimuthal tilt and an elevational tilt of the acoustic beam,
when the ultrasound system is operating in a lung imaging mode, the updating of the scan pattern comprises an adjustment of a combination of an elevational tilt and a translation of an aperture of the two-dimensional array of ultrasound transducers emitting the acoustic beam, and
configures the ultrasound device to obtain a second ultrasound image frame using the updated scan pattern.

2. The ultrasound system of claim 1, wherein the updating of the scan pattern comprises:
configuring the two-dimensional array of ultrasound transducers to obtain a plurality of search image frames, each of the search image frames captured with a different search scan pattern, and
obtaining the updated scan pattern for the second ultrasound image frame by selecting a search scan pattern from the different search scan patterns as the updated scan pattern,
wherein the selected search scan pattern is selected based on the associated search image frame being suitable for a diagnostic action to be performed using the second ultrasound image frame.

3. The ultrasound system of claim 2, wherein the suitability of the search scan pattern is determined based on at least one selected from a group consisting of an amount of the desired anatomy being captured, and a degree of motion.

4. The ultrasound system of claim 2, wherein the obtaining of the plurality of search image frames comprises stepping through a scan pattern sequence of scan patterns.

5. The ultrasound system of claim 4, wherein the stepping through the scan pattern sequence is performed by one selected from a group consisting of:
a full sweep of the scan patterns in the scan pattern sequence,
a coarse sweep to identify a range, followed by a fine sweep in the range, and
a decoupled elevational and azimuthal search through the scan patterns in the scan pattern sequence.

6. The ultrasound system of claim 1, wherein the updating of the scan pattern
is triggered by the at least one first ultrasound image frame not being suitable for a diagnostic action, in absence of a user input.

7. The ultrasound system of claim 1, wherein the updating of the scan pattern comprises:
configuring the two-dimensional array of ultrasound transducers to obtain a first search image frame, captured with a first search scan pattern,
making a first determination that the first search image frame is more suitable for a diagnostic action to be performed than the at least one first ultrasound image frame, and
based on the first determination, obtaining the updated scan pattern for the second ultrasound image frame by selecting the first search scan pattern as the updated scan pattern.

8. The ultrasound system of claim 7, wherein the updating of the scan pattern further comprises:
configuring the two-dimensional array of ultrasound transducers to obtain a second search image frame, captured with a second search scan pattern,
making a second determination that the second search image frame is not more suitable for a diagnostic action to be performed than the second ultrasound image frame, and
based on the second determination, configuring the two-dimensional array of ultrasound transducers to obtain a third ultrasound image frame using the updated scan pattern as used for the obtaining of the second ultrasound image frame.

9. A method for operating an ultrasound system (100), the method, performed by a processor (104) of a smartphone or tablet of the ultrasound system, comprising:
configuring, a two-dimensional array (152) of ultrasound transducers disposed in an ultrasound probe of the ultrasound system to obtain at least one first ultrasound image frame comprising a view of a desired anatomy, using a scan pattern defining an acoustic beam,
automatically updating the scan pattern to optimize the view of the desired anatomy, wherein:
when the ultrasound system is operating in a cardiac imaging mode, the updating of the scan pattern comprises an adjustment of a combination of an azimuthal tilt and an elevational tilt of the acoustic beam,
when the ultrasound system is operating in a lung imaging mode, the updating of the scan pattern comprises an adjustment of a combination of an elevational tilt and a translation of an aperture of the two-dimensional array of ultrasound transducers emitting the acoustic beam, and
configuring the two-dimensional array of ultrasound transducers to obtain a second ultrasound image frame using the updated scan pattern.

10. The method of claim 9, wherein the updating of the scan pattern comprises:
configuring the two-dimensional array of ultrasound transducers to obtain a plurality of search image frames, each of the search image frames captured with a different search scan pattern,
obtaining the updated scan pattern for the second ultrasound image frame by selecting a search scan pattern from the different search scan patterns as the updated scan pattern,
wherein the selected search scan pattern is selected based on the associated search image frame being suitable for a diagnostic action to be performed using the second ultrasound image frame.

11. The method of claim 10, wherein the suitability of the search scan pattern is determined based on at least one selected from a group consisting of an amount of the desired anatomy being captured, and a degree of motion.

12. The method of claim 10, wherein the obtaining of the plurality of search image frames comprises stepping through a scan pattern sequence of scan patterns.

13. The method of claim 12, wherein the stepping through the scan pattern sequence is performed by one selected from a group consisting of:
a full sweep of the scan patterns in the scan pattern sequence,
a coarse sweep to identify a range, followed by a fine sweep in the range, and
a decoupled elevational and azimuthal search through the scan patterns in the scan pattern sequence.

14. The method of claim 9, wherein the updating of the scan pattern is triggered
by the at least one first ultrasound image frame not being suitable for a diagnostic action, in absence of a user input.

15. The method of claim 9, wherein the updating of the scan pattern comprises:
configuring the two-dimensional array of ultrasound transducers to obtain a first search image frame, captured with a first search scan pattern,
making a first determination that the first search image frame is more suitable for a diagnostic action to be performed than the at least one first ultrasound image frame, and
based on the first determination, obtaining the updated scan pattern for the second ultrasound image frame by selecting the first search scan pattern as the updated scan pattern.

## Patentansprüche

1. Ultraschallsystem (100), Folgendes umfassend:
eine Ultraschallvorrichtung (102) mit einer zweidimensionalen Anordnung (152) von Ultraschallwandlern, wobei die Ultraschallvorrichtung als Handgerät oder tragbares Pflaster ausgeführt ist; und
ein Smartphone oder Tablet mit einem Prozessor (104), der:
die Ultraschallvorrichtung konfiguriert, um mindestens einen ersten Ultraschallbild-Frame unter Verwendung eines Abtastmusters zu erhalten, das einen Schallstrahl definiert,
das Abtastmuster automatisch aktualisiert, um eine Ansicht einer gewünschten Anatomie zu optimieren, wobei:
wenn das Ultraschallsystem in einem Herzbildgebungsmodus arbeitet, die Aktualisierung des Abtastmusters eine Anpassung einer Kombination aus einer Azimutneigung und einer Elevationsneigung des Schallstrahls umfasst,
wenn das Ultraschallsystem in einem Lungenbildgebungsmodus arbeitet, die Aktualisierung des Abtastmusters eine Anpassung einer Kombination aus einer Elevationsneigung und einer Translation einer Apertur der zweidimensionalen Anordnung von Ultraschallwandlern umfasst, die den Schallstrahl aussendet, und
die Ultraschallvorrichtung konfiguriert, um einen zweiten Ultraschallbild-Frame unter Verwendung des aktualisierten Abtastmusters zu erhalten.

2. Ultraschallsystem nach Anspruch 1, wobei die Aktualisierung des Abtastmusters Folgendes umfasst:
Konfigurieren der zweidimensionalen Anordnung von Ultraschallwandlern zum Erhalten einer Vielzahl von Such-Bildframes, wobei jeder der Such-Bildframes mit einem unterschiedlichen Such-Abtastmuster erfasst wird, und
Erhalten des aktualisierten Abtastmusters für den zweiten Ultraschallbild-Frame durch Auswählen eines Such-Abtastmusters aus den unterschiedlichen Such-Abtastmustern als das aktualisierte Abtastmuster,
wobei das ausgewählte Such-Abtastmuster basierend darauf ausgewählt wird, dass der zugehörige Such-Bildframe für eine diagnostische Maßnahme geeignet ist, die unter Verwendung des zweiten Ultraschallbild-Frames durchzuführen ist.

3. Ultraschallsystem nach Anspruch 2, wobei die Eignung des Such-Abtastmusters basierend auf mindestens einem aus einer Gruppe bestimmt wird, die aus einem Umfang der erfassten gewünschten Anatomie und einem Bewegungsgrad besteht.

4. Ultraschallsystem nach Anspruch 2, wobei das Erhalten der Vielzahl von Such-Bildframes ein Durchschreiten einer Abtastmuster-Sequenz von Abtastmustern umfasst.

5. Ultraschallsystem nach Anspruch 4, wobei das Durchschreiten der Abtastmuster-Sequenz durch eines aus einer Gruppe durchgeführt wird, die Folgendes umfasst:
einen vollständigen Durchlauf der Abtastmuster in der Abtastmuster-Sequenz,
einen groben Durchlauf zur Identifizierung eines Bereichs, gefolgt von einem feinen Durchlauf in dem Bereich, und
eine entkoppelte Elevations- und Azimutsuche durch die Abtastmuster in der Abtastmuster-Sequenz.

6. Ultraschallsystem nach Anspruch 1, wobei die Aktualisierung des Abtastmusters dadurch ausgelöst wird, dass der mindestens eine erste Ultraschallbild-Frame ohne Benutzereingabe für eine diagnostische Maßnahme nicht geeignet ist.

7. Ultraschallsystem nach Anspruch 1, wobei die Aktualisierung des Abtastmusters Folgendes umfasst:
Konfigurieren der zweidimensionalen Anordnung von Ultraschallwandlern zum Erhalten eines ersten Such-Bildframes, der mit einem ersten Such-Abtastmuster erfasst wird,
Treffen einer ersten Bestimmung, dass der erste Such-Bildframe für eine durchzuführende diagnostische Maßnahme besser geeignet ist als der mindestens eine erste Ultraschallbild-Frame, und
basierend auf der ersten Bestimmung, Erhalten des aktualisierten Abtastmusters für den zweiten Ultraschallbild-Frame durch Auswählen des ersten Such-Abtastmusters als das aktualisierte Abtastmuster.

8. Ultraschallsystem nach Anspruch 7, wobei die Aktualisierung des Abtastmusters ferner Folgendes umfasst:
Konfigurieren der zweidimensionalen Anordnung von Ultraschallwandlern zum Erhalten eines zweiten Such-Bildframes, der mit einem zweiten Such-Abtastmuster erfasst wird,
Treffen einer zweiten Bestimmung, dass der zweite Such-Bildframe für eine durchzuführende diagnostische Maßnahme nicht besser geeignet ist als der zweite Ultraschallbild-Frame, und
basierend auf der zweiten Bestimmung, Konfigurieren der zweidimensionalen Anordnung von Ultraschallwandlern zum Erhalten eines dritten Ultraschallbild-Frames unter Verwendung des aktualisierten Abtastmusters, das für das Erhalten des zweiten Ultraschallbild-Frames verwendet wurde.

9. Verfahren zum Betreiben eines Ultraschallsystems (100), **dadurch gekennzeichnet, dass** das Verfahren von einem Prozessor (104) eines Smartphones oder Tablets des Ultraschallsystems ausgeführt wird, wobei das Verfahren Folgendes umfasst:
Konfigurieren einer zweidimensionalen Anordnung (152) von Ultraschallwandlern, die in einer Ultraschallsonde des Ultraschallsystems angeordnet sind, zum Erhalten mindestens eines ersten Ultraschallbild-Frames, der eine Ansicht einer gewünschten Anatomie umfasst, unter Verwendung eines Abtastmusters, das einen Schallstrahl definiert,
automatisches Aktualisieren des Abtastmusters zum Optimieren der Ansicht der gewünschten Anatomie, wobei:
wenn das Ultraschallsystem in einem Herzbildgebungsmodus arbeitet, die Aktualisierung des Abtastmusters eine Anpassung einer Kombination aus einer Azimutneigung und einer Elevationsneigung des Schallstrahls umfasst,
wenn das Ultraschallsystem in einem Lungenbildgebungsmodus arbeitet, die Aktualisierung des Abtastmusters eine Anpassung einer Kombination aus einer Elevationsneigung und einer Translation einer Apertur der zweidimensionalen Anordnung von Ultraschallwandlern umfasst, die den Schallstrahl aussendet, und
Konfigurieren der zweidimensionalen Anordnung von Ultraschallwandlern zum Erhalten eines zweiten Ultraschallbild-Frames unter Verwendung des aktualisierten Abtastmusters.

10. Verfahren nach Anspruch 9, wobei die Aktualisierung des Abtastmusters Folgendes umfasst:
Konfigurieren der zweidimensionalen Anordnung von Ultraschallwandlern zum Erhalten einer Vielzahl von Such-Bildframes, wobei jeder der Such-Bildframes mit einem unterschiedlichen Such-Abtastmuster erfasst wird,
Erhalten des aktualisierten Abtastmusters für den zweiten Ultraschallbild-Frame durch Auswählen eines Such-Abtastmusters aus den unterschiedlichen Such-Abtastmustern als das aktualisierte Abtastmuster,
wobei das ausgewählte Such-Abtastmuster basierend darauf ausgewählt wird, dass der zugehörige Such-Bildframe für eine diagnostische Maßnahme geeignet ist, die unter Verwendung des zweiten Ultraschallbild-Frames durchzuführen ist.

11. Verfahren nach Anspruch 10, wobei die Eignung des Such-Abtastmusters basierend auf mindestens einem aus einer Gruppe bestimmt wird, die aus einem Umfang der erfassten gewünschten Anatomie und einem Bewegungsgrad besteht.

12. Verfahren nach Anspruch 10, wobei das Erhalten der Vielzahl von Such-Bildframes ein Durchschreiten einer Abtastmuster-Sequenz von Abtastmustern umfasst.

13. Verfahren nach Anspruch 12, wobei das Durchschreiten der Abtastmuster-Sequenz durch eines aus einer Gruppe durchgeführt wird, die Folgendes umfasst:
einen vollständigen Durchlauf der Abtastmuster in der Abtastmuster-Sequenz,
einen groben Durchlauf zur Identifizierung eines Bereichs, gefolgt von einem feinen Durchlauf in dem Bereich, und
eine entkoppelte Elevations- und Azimutsuche durch die Abtastmuster in der Abtastmuster-Sequenz.

14. Verfahren nach Anspruch 9, wobei die Aktualisierung des Abtastmusters dadurch ausgelöst wird, dass der mindestens eine erste Ultraschallbild-Frame ohne Benutzereingabe für eine diagnostische Maßnahme nicht geeignet ist.

15. Verfahren nach Anspruch 9, wobei die Aktualisierung des Abtastmusters Folgendes umfasst:
Konfigurieren der zweidimensionalen Anordnung von Ultraschallwandlern zum Erhalten eines ersten Such-Bildframes, der mit einem ersten Such-Abtastmuster erfasst wird,
Treffen einer ersten Bestimmung, dass der erste Such-Bildframe für eine durchzuführende diagnostische Maßnahme besser geeignet ist als der mindestens eine erste Ultraschallbild-Frame, und
basierend auf der ersten Bestimmung, Erhalten des aktualisierten Abtastmusters für den zweiten Ultraschallbild-Frame durch Auswählen des ersten Such-Abtastmusters als das aktualisierte Abtastmuster.

## Revendications

1. Système à ultrasons (100) comprenant :
un dispositif à ultrasons (102) doté d'un réseau bidimensionnel (152) de transducteurs à ultrasons, le dispositif à ultrasons étant mis en œuvre sous la forme d'un dispositif portatif ou d'un patch portable ; et
un smartphone ou une tablette comprenant un processeur (104) qui :
configure le dispositif à ultrasons pour obtenir au moins une première trame d'image ultrasonore, en utilisant un motif de balayage définissant un faisceau acoustique,
met automatiquement à jour le motif de balayage pour optimiser une vue d'une anatomie souhaitée, dans lequel :
lorsque le système à ultrasons fonctionne en mode imagerie cardiaque, la mise à jour du motif de balayage comprend un réglage d'une combinaison d'une inclinaison azimutale et d'une inclinaison d'élévation du faisceau acoustique,
lorsque le système à ultrasons fonctionne en mode imagerie pulmonaire, la mise à jour du motif de balayage comprend un réglage d'une combinaison d'une inclinaison d'élévation et d'une translation d'une ouverture du réseau bidimensionnel de transducteurs à ultrasons émettant le faisceau acoustique, et
configure le dispositif à ultrasons pour obtenir une deuxième trame d'image ultrasonore en utilisant le motif de balayage mis à jour.

2. Système à ultrasons selon la revendication 1, dans lequel la mise à jour du motif de balayage comprend :
la configuration du réseau bidimensionnel de transducteurs à ultrasons pour obtenir une pluralité de trames d'image de recherche, chacune des trames d'image de recherche étant capturée avec un motif de balayage de recherche différent, et l'obtention du motif de balayage mis à jour pour la deuxième trame d'image ultrasonore en sélectionnant un motif de balayage de recherche parmi les différents motifs de balayage de recherche comme motif de balayage mis à jour,
dans lequel le motif de balayage de recherche sélectionné est sélectionné sur la base de la trame d'image de recherche associée qui convient à une action de diagnostic à effectuer à l'aide de la deuxième trame d'image ultrasonore.

3. Système à ultrasons selon la revendication 2, dans lequel l'adéquation du motif de balayage de recherche est déterminée sur la base d'au moins un élément sélectionné dans un groupe constitué d'une quantité de l'anatomie souhaitée capturée et d'un degré de mouvement.

4. Système à ultrasons selon la revendication 2, dans lequel l'obtention de la pluralité de trames d'image de recherche comprend le défilement d'une séquence de motifs de balayage de motifs de balayage.

5. Système à ultrasons selon la revendication 4, dans lequel le défilement de la séquence de motifs de balayage est effectué par un élément sélectionné dans un groupe constitué de :
un balayage complet des motifs de balayage dans la séquence de motifs de balayage,
un balayage grossier pour identifier une plage, suivi d'un balayage fin dans la plage, et
une recherche découplée en élévation et en azimut à travers les motifs de balayage dans la séquence de motifs de balayage.

6. Système à ultrasons selon la revendication 1, dans lequel la mise à jour du motif de balayage est déclenchée par le fait que l'au moins une première trame d'image ultrasonore n'est pas adaptée à une action de diagnostic, en l'absence d'une entrée utilisateur.

7. Système à ultrasons selon la revendication 1, dans lequel la mise à jour du motif de balayage comprend :
la configuration du réseau bidimensionnel de transducteurs à ultrasons pour obtenir une première trame d'image de recherche, capturée avec un premier motif de balayage de recherche,
la réalisation d'une première détermination selon laquelle la première trame d'image de recherche est plus appropriée pour une action de diagnostic à effectuer que l'au moins une première trame d'image ultrasonore, et
sur la base de la première détermination, l'obtention du motif de balayage mis à jour pour la deuxième trame d'image ultrasonore en sélectionnant le premier motif de balayage de recherche comme motif de balayage mis à jour.

8. Système à ultrasons selon la revendication 7, dans lequel la mise à jour du motif de balayage comprend en outre :
la configuration du réseau bidimensionnel de transducteurs à ultrasons pour obtenir une deuxième trame d'image de recherche, capturée avec un second motif de balayage de recherche,
la réalisation d'une seconde détermination selon laquelle la deuxième trame d'image de recherche n'est pas plus appropriée pour une action de diagnostic à effectuer que la deuxième trame d'image ultrasonore, et
sur la base de la seconde détermination, la configuration du réseau bidimensionnel de transducteurs à ultrasons pour obtenir une troisième trame d'image ultrasonore en utilisant le motif de balayage mis à jour tel qu'utilisé pour l'obtention de la deuxième trame d'image ultrasonore.

9. Procédé de fonctionnement d'un système à ultrasons (100), le procédé, exécuté par un processeur (104) d'un smartphone ou d'une tablette du système à ultrasons, comprenant :
la configuration d'un réseau bidimensionnel (152) de transducteurs à ultrasons disposés dans une sonde à ultrasons du système à ultrasons pour obtenir au moins une première trame d'image ultrasonore comprenant une vue d'une anatomie souhaitée, en utilisant un motif de balayage définissant un faisceau acoustique,
la mise à jour automatique du motif de balayage pour optimiser la vue de l'anatomie souhaitée, dans lequel :
lorsque le système à ultrasons fonctionne en mode imagerie cardiaque, la mise à jour du motif de balayage comprend un réglage d'une combinaison d'une inclinaison azimutale et d'une inclinaison d'élévation du faisceau acoustique,
lorsque le système à ultrasons fonctionne en mode imagerie pulmonaire, la mise à jour du motif de balayage comprend un réglage d'une combinaison d'une inclinaison d'élévation et d'une translation d'une ouverture du réseau bidimensionnel de transducteurs à ultrasons émettant le faisceau acoustique, et
la configuration du réseau bidimensionnel de transducteurs à ultrasons pour obtenir une deuxième trame d'image ultrasonore en utilisant le motif de balayage mis à jour.

10. Procédé selon la revendication 9, dans lequel la mise à jour du motif de balayage comprend :
la configuration du réseau bidimensionnel de transducteurs à ultrasons pour obtenir une pluralité de trames d'image de recherche, chacune des trames d'image de recherche étant capturée avec un motif de balayage de recherche différent,
l'obtention du motif de balayage mis à jour pour la deuxième trame d'image ultrasonore en sélectionnant un motif de balayage de recherche parmi les différents motifs de balayage de recherche comme motif de balayage mis à jour,
dans lequel le motif de balayage de recherche sélectionné est sélectionné sur la base de la trame d'image de recherche associée qui convient à une action de diagnostic à effectuer en utilisant la deuxième trame d'image ultrasonore.

11. Procédé selon la revendication 10, dans lequel l'adéquation du motif de balayage de recherche est déterminée sur la base d'au moins un élément sélectionné dans un groupe constitué d'une quantité de l'anatomie souhaitée capturée et d'un degré de mouvement.

12. Procédé selon la revendication 10, dans lequel l'obtention de la pluralité de trames d'image de recherche comprend le défilement d'une séquence de motifs de balayage de motifs de balayage.

13. Procédé selon la revendication 12, dans lequel le défilement de la séquence de motifs de balayage est effectué par un élément sélectionné dans un groupe constitué de :
un balayage complet des motifs de balayage dans la séquence de motifs de balayage,
un balayage grossier pour identifier une plage, suivi d'un balayage fin dans la plage, et
une recherche découplée en élévation et en azimut à travers les motifs de balayage dans la séquence de motifs de balayage.

14. Procédé selon la revendication 9, dans lequel la mise à jour du motif de balayage est déclenchée par le fait que l'au moins une première trame d'image ultrasonore n'est pas adaptée à une action de diagnostic, en l'absence d'une entrée utilisateur.

15. Procédé selon la revendication 9, dans lequel la mise à jour du motif de balayage comprend :
la configuration du réseau bidimensionnel de transducteurs à ultrasons pour obtenir une première trame d'image de recherche, capturée avec un premier motif de balayage de recherche,
la réalisation d'une première détermination selon laquelle la première trame d'image de recherche est plus appropriée pour une action de diagnostic à effectuer que l'au moins une première trame d'image ultrasonore, et
sur la base de la première détermination, l'obtention du motif de balayage mis à jour pour la deuxième trame d'image ultrasonore en sélectionnant le premier motif de balayage de recherche comme motif de balayage mis à jour.
